# EUROPEAN PATENT SPECIFICATION

(11) **EP 3 027 750 B1**
(45) Date of publication and mention of the grant of the patent: **03.04.2019**
(21) Application number: 14748154.3
(22) Date of filing: 30.07.2014
(51) Int. Cl.: C12N 11/04, C12N 11/14, B01J 13/14, B82Y 5/00, B82Y 30/00, A61K 38/00, C12N 9/96

(54) **BIOCATALYTICAL COMPOSITION**
BIOKATALYTISCHE ZUSAMMENSETZUNG
COMPOSITION BIOCATALYTIQUE

(30) Priority: 30.07.2013 EP 13178504
(43) Date of publication of application: 08.06.2016
(73) Proprietor: Inofea AG, 4057 Basel (CH)
(72) Inventor: SHAHGALDIAN, Patrick, 68300 Saint Louis (FR); CORRERO-SHAHGALDIAN, Maria Rita, 68300 Saint Louis (FR); CUMBO, Alessandro, 4056 Basel (CH); CORVINI, Philippe Francois-Xavier, 68220 Leymen (FR)
(74) Representative: Latscha Schöllhorn Partner AG
(86) International application number: PCT/EP2014/066365
(87) International publication number: WO 2015/014888

(56) References cited:
- WO-A2-93/07263
- WO-A2-2005/056808
- US-B1- 6 268 329
- YONG-QING XIA ET AL: "Protein recognition onto silica particles using chitosan as intermedium substrate", JOURNAL OF BIOMEDICAL MATERIALS RESEARCH PART A, vol. 90A, no. 2, 28 May 2008 (2008-05-28), - 1 August 2009 (2009-08-01), pages 326-332, XP055099686, ISSN: 1549-3296, DOI: 10.1002/jbm.a.32084
- MATEO ET AL: "Improvement of enzyme activity, stability and selectivity via immobilization techniques", ENZYME AND MICROBIAL TECHNOLOGY, STONEHAM, MA, US, vol. 40, no. 6, 29 March 2007 (2007-03-29) , pages 1451-1463, XP022003968, ISSN: 0141-0229, DOI: 10.1016/J.ENZMICTEC.2007.01.018
- ULF HANEFELD ET AL: "Understanding enzyme immobilisation", CHEMICAL SOCIETY REVIEWS, vol. 38, no. 2, 9 December 2008 (2008-12-09), - 1 January 2009 (2009-01-01), page 453, XP055099160, ISSN: 0306-0012, DOI: 10.1039/b711564b cited in the application
- DEAN BRADY ET AL: "Advances in enzyme immobilisation", BIOTECHNOLOGY LETTERS, SPRINGER NETHERLANDS, DORDRECHT, vol. 31, no. 11, 10 July 2009 (2009-07-10) , pages 1639-1650, XP019746235, ISSN: 1573-6776, DOI: 10.1007/S10529-009-0076-4 cited in the application
- GALLIKER P ET AL: "Laccase-modified silica nanoparticles efficiently catalyze the transformation of phenolic compounds", JOURNAL OF COLLOID AND INTERFACE SCIENCE, ACADEMIC PRESS, NEW YORK, NY, US, vol. 349, no. 1, 16 May 2010 (2010-05-16), - 1 September 2010 (2010-09-01), pages 98-105, XP027113832, ISSN: 0021-9797 [retrieved on 2010-05-16]
- CHIORCEA-PAQUIM A M ET AL: "AFM nanometer surface morphological study of in situ electropolymerized neutral red redox mediator oxysilane sol-gel encapsulated glucose oxidase electrochemical biosensors", BIOSENSORS AND BIOELECTRONICS, ELSEVIER BV, NL, vol. 24, no. 2, 11 April 2008 (2008-04-11) , - 15 October 2008 (2008-10-15), pages 297-305, XP023439562, ISSN: 0956-5663, DOI: 10.1016/J.BIOS.2008.04.001 [retrieved on 2008-04-11]
- SANJIB BHATTACHARYYA ET AL: "Polymer-coated mesoporous silica nanoparticles for the controlled release of macromolecules", ACTA BIOMATERIALIA, vol. 8, no. 9, 9 June 2012 (2012-06-09), - 1 September 2012 (2012-09-01), pages 3429-3435, XP055099688, ISSN: 1742-7061, DOI: 10.1016/j.actbio.2012.06.003
- ALESSANDRO CUMBO ET AL: "A synthetic nanomaterial for virus recognition produced by surface imprinting", NATURE COMMUNICATIONS, vol. 4, 19 February 2013 (2013-02-19), pages 1503-1503, XP55137331, ISSN: 2041-1723, DOI: 10.1038/ncomms2529
- Alessandro Cumbo ET AL: "A synthetic nanomaterial for virus recognition produced by surface imprinting", Nature Communications, vol. 4, 1 January 2013 (2013-01-01), pages 1503-1503, XP55137331, ISSN: 2041-1723, DOI: 10.1038/ncomms2529
- SHIOMI T ET AL: "A method for the molecular imprinting of hemoglobin on silica surfaces using silanes", BIOMATERIALS, ELSEVIER SCIENCE PUBLISHERS BV., BARKING, GB, vol. 26, no. 27, 1 September 2005 (2005-09-01), pages 5564-5571, XP025280804, ISSN: 0142-9612, DOI: 10.1016/J.BIOMATERIALS.2005.02.007 [retrieved on 2005-09-01]
- M. RITA CORRERO ET AL: "Enzyme Shielding in an Enzyme-thin and Soft Organosilica Layer", ANGEWANDTE CHEMIE INTERNATIONAL EDITION, vol. 55, no. 21, 9 April 2016 (2016-04-09) , pages 6285-6289, XP055351976, DE ISSN: 1433-7851, DOI: 10.1002/anie.201600590
- DATABASE MEDLINE [Online] US NATIONAL LIBRARY OF MEDICINE (NLM), BETHESDA, MD, US; 22 February 2002 (2002-02-22), EISENMESSER ELAN ZOHAR ET AL: "Enzyme dynamics during catalysis.", Database accession no. NLM11859194 & EISENMESSER ELAN ZOHAR ET AL: "Enzyme dynamics during catalysis.", SCIENCE (NEW YORK, N.Y.) 22 FEB 2002, vol. 295, no. 5559, 22 February 2002 (2002-02-22), pages 1520-1523, ISSN: 1095-9203
- ERICKSON HAROLD P: "Size and Shape of Protein Molecules at the Nanometer Level Determined by Sedimentation, Gel Filtration, and Electron Microscopy", BIOLOGICAL PROCEDURES ONLINE, BIOLOGICAL PROCEDURES ONLINE, WATERLOO, CA, vol. 11, no. 1, 15 May 2009 (2009-05-15), pages 32-51, XP021088900, ISSN: 1480-9222, DOI: 10.1007/S12575-009-9008-X
- GLAD M ET AL: "Use of silane monomers for molecular imprinting and enzyme entrapment in polysiloxane-coated porous silica", JOURNAL OF CHROMATOGRAPHY A, ELSEVIER, AMSTERDAM, NL, vol. 347, 1 January 1985 (1985-01-01), pages 11-23, XP026474888, ISSN: 0021-9673, DOI: 10.1016/S0021-9673(01)95465-2 [retrieved on 1985-01-01]

## Description

The present invention relates to the protection of proteins and protein type compounds. More particularly, the present invention relates to methods and compositions which allow proteins and protein type compounds to be protected, e.g. under adverse conditions.

Proteins and protein-type compounds such as enzymes are frequently needed, e.g. in industrial applications, diagnostics for therapeutic use and so forth.

In these applications, it cannot be guaranteed that the conditions under which e.g. an enzyme is to be used will be those where the activity of the enzyme will be at a maximum. Rather, it is to be expected that the actual conditions deviate significantly from optimum conditions, e.g. with respect to temperature, presence of pollutants, solvents asf. Thus, frequently, the proteins and protein-type compounds are subject to substantive stress in use or prior to use, e.g. during storage. This may adversely affect their activity or use, particular as proteins or protein-type compounds frequently are expensive and hence loss of active material or activity might cause a significant increase in costs.
It has been suggested before to immobilize enzymes on the surface of a carrier and to protect them with a layer of protective material so as to generate a biocatalytical composition with increased resistance to various types of stresses.

In this respect, it has been stated that the use of immobilized enzymes in industrial applications guarantees their removal and consequently simplifies the design of reactor and the control of reaction (C. Mateo et al.). A large number of enzyme immobilization and protection methods have been developed (D. Brady and J. Jordaan). Those methods present advantages such as possible reusability of the immobilized enzyme, protection of the enzyme from mechanical (bubbles), chemical (pH changes, chaotropic agents) and biological (proteases) stresses but also disadvantages like enzyme leakage from the support, loss of the protein tertiary/active structure, restriction of the substrate diffusion (R.C. Rodrigues *et al.*) and in case of porous material (e.g. sephabeads) clogging of the carrier. Moreover, to allow the anchoring of the enzyme to the support, the enzyme is occasionally genetically modified (H. R. Luckarift *et al.).*

Hanefeld *et al.,* published a review about the understanding of enzyme immobilization. This work focuses on the importance of the enzyme (*e.g*. size, stability, need of additives) and carrier (chemical and mechanical stability, porosity) characteristics as well as of reaction factors such as diffusion limitations and enzyme inhibition to name but a few (U. Hanefeld *et al.*).
Hartmann and Jung reported how the immobilization of enzymes on mesoporous supports enhances their operational stability and enables their reuse for continuous processes. Hilterhaus *et al*., reported on the immobilization of several enzymes (endoglucanase, decarboxylase and lipase) onto Sepabeads supports and on the benefit of repetitive use in a bubble column reactor as well as in a stirred tank reactor.
In 2005, Naik *et al.,* filed a patent application for a method of enzyme immobilization by biosilicification. The authors described that the immobilization occurred by mixing the enzyme, the silica precursor, and the silaffin peptide; the resulting material maintained enzyme activity with improved temporal stability.

Recently, Werchant filed a patent for a method describing the preparation of immobilized enzyme. The system consists of a network composed of enzyme, polymer, and crosslinker. The covalently immobilized enzyme system retained stable activity when dried and stored at ambient conditions (S.A. Werchant).

US No. 2012 0 149 082 A1 discloses the synthesis of crosslinked enzyme-silica aggregates; in particular for the immobilization of laccase, lipase, protease, esterase, oxynitrilase, nitrilase, aminoacylase, penicillin acylase, lyase, oxidase and reductase. The complex is prepared by taking up the enzyme in a solvent, precipitating the enzyme and adding an alkoxysilane and crosslinking agent such as glutaraldehyde.

Ostaszewski filed a patent for a method concerning the immobilization of levostatin esterase enzyme on an aminopropyl-silanized silica gel activated by cyanuric chloride.

US 7 642 077 B2 discloses another method for enzyme or cell immobilization by co-precipitation with silica and/or organosilicate solution through the action of an organic template molecule namely a polyamine or a polypeptide.

Chung *et al.,* filed a patent for a cascade enzyme-linked immunosorbent assay using magnetic microparticles and tosyl-functionalized magnetic silica naparticles for the immobilization of the target antigen and the antigen-specific secondary antibody, respectively (S. J. Chung *et al.).*

The use of mesoporous-silica binding histidine-tagged protein has been filed in the US 2010/0264188A1. The mesopours-silica is first made magnetic by Fe adsorbtion, and then coated with Ni to enable the binding of a specific protein/enzyme labeled with histidine.

Auger *et al.,* disclosed a method for the incorporation of biological macromolecules (at least one protein or nucleic acid) inside porous silica nanoparticles (30-40 nm) functionalized by groups setting up an ionic and/or hydrogen non-covalent bonds with the target molecule (J.H. Chang *et al.,* A. Auger *et al.*)*.*

WO2011 06 0129 A1 discloses a method to covalently immobilize and protect enzymes in a thermally responsive polymer shell. The covalent grafting (e.g. vinyl group attachment) of the enzyme allows the covalent binding of the protein to the polymer. The polymer shell comprises at least one thermoresponsive polymer such as poly(N-isopropylacrylamide), poly(isopropyl-N-vinylpyrrolidone), and/or N-isopropylacrylamide, and/or polystyrene polymer. The encapsulated enzyme is stable at temperature higher than 30 °C and can be used for chemical remediation, drug delivery, wound healing and protein therapy (A. Auger *et al.).*

Recently, Lant filed a patent for a method of treating textile with an aqueous solution containing protected particles containing lipid esterase. In more detail, the core of the protected particles comprises the enzyme and the substrate and it is surrounded by delayed-release coating layers (containing polyethylene glycol, polyvinyl alcohol or hydroxypropyl methyl cellulose) that can also contain the substrate (N. J. Lant *et al.).*

Beside the enzyme protection, many important applications of anchoring proteins onto supports including the fabrication of functional protein microarrays and biosensors have acquired great importance (L.S. Wong *et al.).*

WO 2012/142625A2 discloses a method for the fabrication of a "nanoparticle-device" that can be used for delivering of substances into living target tissue, preferably tumor tissue. A nanoparticle device includes a shell structure made by an internal and an external layer. The internal layer contains the deliverable substance and possesses holes; the external layer, that is made by a porous material, seals the holes. In controlled conditions the external layer degrades and allows the delivery of the substance contained in the nanoparticle-device (L.S. Wong *et al.).*

β-galactosidase is an enzyme (hydrolase) that catalyzes the hydrolysis reaction of β-galactosides into monosaccharides. For instance, it allows the hydrolysis of lactose into glucose and galactose.

Because of its tremendous importance in the dairy industry, the immobilization of β-galactosidase has been extensively investigated and different strategies have been developed (S. C. Esener *et al.).*

Already in 1981 a patent about immobilization of lactase on solid support has been filed by Sumitomo Chemical Company (EP 0 026 672 A2). This document discloses a method for immobilizing active lactase on a macroporous amphoteric phenol-formaldehyde type ionexchange resin (Q. Husain).

In 1998, Giacomini *et al.* reported the covalent immobilization of β-galactosidase from *K. lactis* onto two different porous supports: an inorganic carrier silane-coated (CPC-silica) and agarose (C. Giacomini *et al.).* CPC-silica was activated with glutaraldehyde while agarose was activated via a cyanylating agent. Although higher amounts of β-galactosidase were immobilized on the activated CPC-silica compared to the other support, only 34 % of the enzymatic activity was expressed. The authors explain this result as enzyme inactivation occurring during immobilization.

Later, Tanriseven and Dogan showed that β-galactosidase from *Asperigillus oryzae* immobilized on alginate and gelatin fibers, hardened with glutaraldehyde, maintained 56 % of its initial activity.

Mammarella and Rubiolo reported on the entrapment of β-galactosidase from *Kluyveromyces fragilis* in alginate-carrageenan gels beads. The presence of carrageenan had a favorable influence on the enzyme-catalyzed reaction because the gel is formed within K⁺ ions, which increase the activity of the enzyme. Nevertheless, a quite relevant enzyme leakage from the support was observed during continuous reactions.

Hydrophilic carriers such as cellulose, agarose, chitosan, dextran, alginate, gelatin, and collagen, have been used as immobilized-enzyme support with significant activity retention (E.J. Mammarella and A.C. Rubiolo, S. Rejikumar and S. Devi). However, these materials are not always suitable for immobilized enzyme applications as they can swell and/or degrade under operational conditions or in the presence of microbial organisms.

Fibrous polymeric materials cross-linked with glutaraldehyde have also been used for enzyme immobilization but their use is characterized by low immobilization efficiency and small enzyme activity. In order to produce galacto-oligosaccharides (GOS), β-galaatosidase from *Aspergillus oryzae* was covalently immobilized on cotton cloth modified with tosyl chloride (N. Albayrak and S.T. Yang). The authors reported that the immobilized enzyme has a half-life of 50 days at 50 °C and more than 1 year at 40 °C. The procedure for preparing the cotton fibers is tedious and involves the use of organic chemicals. In order to overcome those disadvantages, a new strategy of cotton modification with polyethyleneimine was developed (N. Albayrak and S. T. Yang). The main drawback of this technique remains the difficulty of production in a large scale.

Mateo *et al.* measured the activity of β-galactosidase from *K. lactis* immobilized onto different supports: an epoxy-boronate resin (Eupergit), glyoxyl-agarose, glutaraldehyde-agarose and glutaraldehyde-Eupergit. In general, the activity of the immobilized enzyme was 10 % higher than the free enzyme. Nevertheless, epoxide activated supports presented the main limitation of requiring a superficial modification with adsorbing groups that allow the enzyme to be adsorbed on the support. These interactions may alter the structure of the catalyst and produce changes in the enzyme features and performances.

In 2000 Ladero *et al.* described the hydrolysis of lactose by a β-galactosidase from *K. fragilis* covalently immobilized on a commercial silica-alumina support silanized with APTES (3-aminopropyltriethoxysilane) and modified with glutaraldehyde. The immobilized enzyme presented a catalytic activity 50 % inferior to that of the free enzyme. Additionally, the activity was limited in a range of temperature between 5°C and 40 °C which may not be suitable for milk treatment.

Betancor *et al.* in 2007 reported a new strategy for creating a three-dimensional network of silica nanospheres containing entrapped β-galactosidase from *E. coli* attached to a silicon support. The immobilized β-galactosidase was stable and retained more than 80 % of its initial activity after 10 days at 24°C. Even if successful, this approach presents the main disadvantage of tedious preparation procedure and difficulty of production in large scale.
One of the main disadvantages of the enzyme immobilization on silicate materials is the low porosity of the silica-gel or the molecules/enzyme packing within the mesopore channels, thereby resulting in a catalytic activity lower than that of free enzymes (Y. Kuwahara *et al).*

β-galactosidase from *A oryzae* was entrapped in lens-shaped PVA (polyvinyl alcohol) hydrogel capsules, (diameter 3-4 mm, thickness 200-400 µm) for the hydrolysis of lactose. The enzyme kept only 32 % of its original activity. Additionally, after enzyme immobilization a general decrease of β-galactosidase affinity for its substrate was observed. The authors explained it through possible structural changes of the enzyme after interaction with the matrix and probable restriction of the substrate diffusion through the hydrogel (Z. Grosova *et al.).*

Neri *et al.* reported on the entrapment of β-galactosidase from *K. lactis* in glutaraldehyde activated magnetic beads of a polyvinyl alcohol (PVA) and polysiloxane (POS). The immobilized enzyme retained about half of its initial activity after being reused 20 times. However, the catalytic efficiency of the immobilized enzyme was only 12 % compared to that found for the native enzyme. This reduced catalytic activity can be due to enzyme conformational changes induced by the support, or to steric difficulties or limited diffusion of the substrate.

Although those methods are quite interesting and promising, their main limitation consists in the deficiency of anchoring sites for the enzymes thus in the possible leaking of the catalyst from the support, or in the uncorrected three-dimensional arrangement of the enzyme in its microenvironment. Additionally, most of those methods suffer from the disadvantage of being expensive, thus inappropriate for industrial-large scale production.

US 2010 196 985 A1 discloses a method for covalently linking lactase to a hydrophobic functionalized polymeric support, namely a food packaging material (e.*g*. poly(ethylene), poly(ethylene vinyl acetate), polystyrene/acrylic acid copolymer). Although this invention allows overcoming general drawbacks of enzyme immobilization techniques such as catalyst leakage, carrier stability and cost it has the main limit of enzyme reusability.

Furthermore, from WO 2005/ 056808 A2, the immobilization of biocatalysts by template-directed silicate precipitation is known. The document suggests a biocomposite where one or more biocatalysts and silica or organosilicates are co-precipitated.

From EP 2 431 742 A1, the preparation of a molecular Recognition Element is known. It is suggested to bind a template to a surface of carrier material, to then initiate polymerization of recognition material, to stop the polymerization and to release the template from the polymerized recognition material. It is stated that a preferred polymerization can be based on a poly-condensation of silica precursors such as tri-alkoxy-silane and tetra-alkoxy-silane under aqueous conditions.

From WO 93/07263, an enzyme-containing granule is known, comprising a core having a water-soluble or dispersible material coated with a vinyl polymer or copolymer, an enzyme layer comprising one or more enzyme and a vinyl polymer or copolymer and an outer coating comprising a vinyl polymer or copolymer.

From US 6,268,329B12, an enzyme-containing granule is known, wherein an enzyme-containing core is provided comprising a coating with substantive amounts of water-soluble coatings. It is suggested to use this granule in a detergent composition.

From S. BHATTACHARYYA et al., "Polymer-coated mesoporous silica nanoparticles for the controlled release of macromolecules" are known. BHATTACHARYYA suggests to use a mesoporous silica nanosphere prepared for immobilization of trypsin inhibitor used as a "model protein" and to then covalently attach a thin layer of PEG-amine to the surface of the of trypsin inhibitor -loaded carrier, the PEG having a molecular weight of 3kDa and hence composed of approximately 68 ethylene glycol units.

From Yong-Qing Xia et al. "Protein recognition onto silica particles using chitosan as inter-medium substrate" a molecular imprinting method is known to prepare twice-coated silica particles with specific recognition sites for hemoglobin. Chitosan was used as an intermedi-um to be coated on silica particles via phase inversion process, and the abundance of exposed amine groups were active sites for introducing aldehyde groups. After hemoglobin was covalently immobilized by forming imine bonds with the aldehyde groups, acrylamide was then polymerized onto chitosan-coated silica particles to form the recognition sites.

According to P. Galliker et al. " Laccase-modified silica nanoparticles efficiently catalyze the transformation of phenolic compounds" a system based on laccase-modified silica nanoparticles has been tested for its ability to degrade a major endocrine disrupting chemical, 4,4'-isopropylidenediphenol (bisphenol A).For this, nano- particles have been produced using the Stoeber method and characterized using scanning electron microscopy, dynamic light scattering and □-potential measurements. Introduction of primary amino groups at the surface of these particles has been effected using an organo-silane (amino-propyl-triethoxysilane). The use of glutaraldehyde as bi- functional coupling agent has allowed conjugation of a laccase from *Coriolopsis polyzona* at the surface of the nanoparticles, as monitored by measuring the amount of proteins coupled and the □-potential of the produced nanoparticles. The oxidative activity of the so-produced bio-conjugate was tested using radioactive labeled bisphenol A. It has been demonstrated that even if a decrease of the specific catalytic activity of the immobilized enzyme is measured, the activity of the bio-conjugate remains compatible with the application of these systems to the transformation of phenolic pollutants.

According to A.-M. Chiorcea-Paquim et al. "AFM nanometer surface morphological study of in situ electropolymerized neutral redox mediator oxysilane sol-gel encapsulated glucose oxidase electrochemical biosensors ", four different silica sol-gel films: methyltrimethoxysilane (MTMOS), tetraethoxysilane (TEOS), 3-aminopropyltriethoxysilane (APTOS) and 3-glycidoxypropyl-trimethoxysilane ( GOPMOS) assembled onto highly oriented pyrolytic graphite (HOPG) were characterized using atomic force microscopy (AFM), due to their use in the development of glucose biosensors. The chemical structure of the oxysilane precursor and the composition of the sol-gel mixture were stated to influence the roughness, the size and the distribution of pores in the sol-gel films, which is relevant for enzyme encapsulation. The GOPMOS sol-gel film is stated to fulfill all morphological characteristics required for good encapsulation of the enzyme, due to a smooth topography with an allegedly very dense and uniform distribution of only small, 50 nm diameter, pores at the surface. APTOS and MTMOS sol-gel films are stated to develop small pores together with large ones of 300-400 nm that allow the leakage of enzymes, while the TEOS film is reported to form a rough and incomplete network on the electrode, less suitable for enzyme immobilization. The AFM results are stated to explain the variation of the stability in time, sensitivity and to limit of detection obtained with different oxysilane sol-gel encapsulated glucose oxidase biosensors with redox mediator.

There is therefore an unmet need for providing an economical and easy-to-use system for protecting proteins or protein-type compounds, particularly enzymes, against unfavorable influences that may inactivate or denaturize the protein or enzyme. Further, there is a special need for a protection system, which allows for use of the protein or enzyme in commercial large-scale applications and for reutilization of the protein or enzyme.

This need is satisfied within the scope of the present invention by the provision of a composition and a method as defined by the features of independent claims. Preferred embodiments are disclosed herein and/or are subject of the dependent claims.

Hence, what is suggested is inter alia a method of producing a composition, the composition comprising at least a solid carrier, a functional constituent, selected from a protein and a protein-type compound, and a protective layer for protecting the functional constituent, by embedding the functional constituent at least partially, wherein the method comprises the steps that first the at least one functional constituent is immobilized on the surface of the solid carrier and then the protective layer for protecting the functional constituent by at least partially embedding the functional constituent, is built with building blocks at least part of which are monomers capable of interacting with both each other and the immobilized functional constituent.

This method allows for a very good protection of the functional constituent. The good results obtainable according to the invention are believed to be due to the following reasons. The monomers forming the protective layer will be capable to interact with both each other and the functional constituent; interaction of the monomers with each other leads to a self-assembly and hence a polymer building reaction and so a polymer is created around the functional constituent the monomers are closely arranged around the functional constituent due to the additional interaction therewith. It will be noted that the interaction of the monomers with each other need not be the same kind of interaction as the interaction of the monomers with the functional constituent; nor is it necessary that such interactions start or become noticeable at the same time. Usually, the monomers forming the protective layer will interact with the functional constituent prior to interaction with each other; the interaction with each other usually will be a reaction of the monomers with each other that will lead to the formation of a polymer around the functional constituent while at the same time, that is while forming the polymers, the monomers are (or remain) closely arranged around the functional constituent due to the additional interaction therewith. Such close arrangement of the building blocks around the functional constituent to be protected will not occur if long polymers are used because for these, the respective functional groups capable of interaction with the functional constituent e.g. via non-covalent binding cannot be expected to be at the right position. Hence, encapsulation according to the invention will be tight. Given that the encapsulation is tight, the functional constituent will be protected better, e.g. because the conformation of the functional constituent will be better maintained.

Embedding will be such that (at least the vast majority or large fraction of) the functional constituent will remain embedded until use, preferably until end of use, and is neither intended to be washed out nor will washing out take place during intended use. Hence, removal of the functional constituent is not intended and should indeed be avoided. According to the present invention, maintaining a mere imprint of the functional constituent washed out from a layer serving merely as a recognition layer releasing the imprinted material prior use is neither intended nor sufficient. In contrast to known releasing recognition layers, the protection layer of the present invention may thus be considered a functional constituent retaining protection layer, retaining said functional constituent during use.

A tight encapsulation helps in retaining. In this context, it should be noted that the term "tight" as used herein may not only be used to refer to a close spatial relationship but that activity of the functional constituent can be used as a measure for tightness, in particular the activity of the functional constituent after prolonged use under certain conditions.

Furthermore, as the embedding layer is built after immobilization and hence after the functional constituent is already on the surface, there need not be protective material below the functional constituent. This is in stark contrast to a co-precipitation, where substantive amounts of protective material will be underneath the functional constituent and hence between the functional constituent and the carrier. Accordingly, the building of the protective layer can be controlled more precisely and a layer with specific properties is more easily obtained.

In a preferred embodiment, the present invention relates to the method as disclosed herein for producing the composition according to the present invention, wherein the thickness of the porous nano-environment is controlled by stopping the self-assembly reaction of the protective material at a specific time point to obtain a protective layer with a desired thickness.

As the thickness of the layer may be one of the specific properties controlled more precisely, burying large amounts of functional components (constituents) too deep within the layer to be reached by target molecules can be avoided hence making better use of the functional constituents used in the production of the composition. The control over thickness thus gives the possibility of obtaining a final protective layer that has about the size of the larger enzyme axis. Such a layer will be a homogeneous layer where all enzyme present in the protective layer is active in the same way.

Accordingly, the method according to the invention preferably will relate to predefining a target size for the protective layer. This predefining of a target size comprises predefining a target thickness, so that the polymerization of the protective material on the surface of the carrier material is stopped when the polymerized protective material has reached a thickness which essentially equals the predefined target thickness. By controlling the thickness of the polymerized protective material, the size of the protective layer embedding the protein or protein-type compound immobilized at the surface of a solid carrier can conveniently be adjusted and optimized for an intended application.

Particularly, by controlling the thickness of the polymerized protective material, the growth of the protective layer may be controlled and adjusted in a range from 1 to 100 nm, 1 nm to 50 nm, 1 nm to 30 nm, 1 nm to 25 nm, 1 nm to 20 nm, 1 nm to 15 nm, preferably 5 nm to 15 nm. Within these ranges, an accuracy level of the growth of the polymerized protective material may be in a range from 1 to 10 nm, from 1 nm to 5 nm, from 1 nm to 4 nm, from 1 nm to 3 nm, from 1 nm to 2 nm, preferably 1 nm. The thickness may be checked using a microscope such as scanning electron microscope (SEM), transmission electron microscopy (TEM), scanning probe microscopy (SPM) or light scattering methods. For example, as it is known in the art, SEM is a type of electron microscope that images a surface of a sample by scanning it with a high-energy beam of electrons in a raster scan pattern. The electrons interact with the atoms that make up the sample producing signals that contain information about the surface's topography (e.g. topography of polymerized protective material), composition and other properties such as electrical conductivity. The way for carrying out such a kind of microscopy for the purpose of analysis is well known to the skilled person.

Stopping the polymerization of the protective material on the surface of the carrier material, when the thickness of the polymerized protective material essentially equals that of the predefined target thickness, allows a precise control of the size of the protective layer. In this context, also a growth kinetic may be preliminarily determined for the growth of the protective layer in terms of thickness of the protective material to be polymerized in a time-dependent manner for given conditions. The results of this determination may then be used to stop the polymerization reaction once the polymerized protective material has reached the predefined layer thickness.

In one aspect, predefining the target thickness of the polymerized protective material comprises predefining a target duration for the polymerization reaction under given reaction conditions. The polymerization of the protective material on the surface of the carrier material is then performed under these conditions and stopped at the predefined time at which the polymerization of the protective material on the surface of the carrier material was determined to essentially equal the predefined target thickness. The term "conditions" in this context relates to parameters which determine the growth of the protective material. In particular, it may relate to the (initial) concentration and composition of the monomeric building blocks used in the protective material, the polymerization temperature, pressure and/or humidity. It will be understood that where reference is made to "interaction of monomers with each other" and where such interaction is a polymerization reaction, the interaction of monomers will also include interaction of monomers with intermediate products obtained during polymerization.

The above procedure allows precisely controlling the thickness of the polymerized protective material, and thus the thickness of the protective layer, particularly the thickness of the protective layer around the immobilized protein or protein-type compound embedded in the protective material. For example, by controlling the thickness of the self-assembled protective material, the activity of an immobilized enzyme embedded in said protective material may be selectively modulated. In this context, a growth kinetic of the polymerized protective material, which takes into account the polymerization duration of the protective material to be polymerized for given conditions and the activity of the enzyme dependent on the thickness of the self-assembled protective material, may be preliminary determined. As a result of this determination, the polymerization reaction may be stopped, once the preferred enzyme activity is reached, which is dependent on the thickness of the layer. For example, the polymerization may be stopped once the balance between enzyme activity and enzyme resistance to stress is optimal.

It should be noted that while it is not necessary to use a 100% pure, dimer- and oligomer free monomer composition or mixture for the method of the present invention, the percentage of monomers used as building blocks should as a general rule be very high in order to obtain good results. Where more building block are in the form of monomers, that is, where a higher percentage of the building blocks is constituted by monomers, the fine and precise self-sorting and organization of the monomers around the enzyme or other immobilized functional constituent is improved, resulting in a higher chance that the resulting protective performances of the embedding layer is particularly effective. When dimers or oligomers are used, the final protective layer would be less tightly wrapped around the enzyme, therefore less effective protective performances has to be expected.

However, commercially available monomers usually will suffice the needs of the present invention. It can be expected that for these more than 80% of the building blocks will be in the form of monomers, and typically more than 95% of the building blocks will be in the form of monomers.

It is advantageous if the monomers used are further capable of interacting with the surface of the solid carrier, as this will ensure that the protective layer binds to the carrier as well, preventing that the protective layer is not tight enough.

Frequently, prior to immobilizing the functional constituent on the surface of the solid carrier, the solid carrier is modified to improve immobilization of the functional constituent on the surface.

So, in a further embodiment the present invention relates to a method as disclosed herein for producing the composition according to the present invention, wherein the surface of said solid carrier as disclosed herein is modified to introduce at least one molecule as anchoring point. Said at least one molecule used as anchoring point may be further modified by inducing a chemical reaction of the at least one molecule as anchoring point with a bi-functional cross-linker. In particular, said anchoring point is an amine moiety; more particularly amino-silane, more particularly 3-aminopropyltriethoxysilane (APTES). The at least one protein or protein-type compound of interest as disclosed herein and, optionally, the at least one optional molecule as disclosed herein is coupled at the surface of the modified carrier through the free active functional group of the bi-functional cross-linker. In particular, said bi-functional cross-linker is glutaraldehyde. Other examples of bi-functional cross-linker are disuccinimidyl tartrate, bis[sulfosuccinimidyl] suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, activated sulfhydryls (e.g. sulfhydryl-reactive 2-pyridyldithio).
In a specific embodiment, the present invention relates to the method as disclosed herein for producing the composition according to the present invention, wherein the free active functional group of the bifunctional cross-linker is an aldehyde, carboxylic acid, imidoester, and/or aryl halid.
In another specific embodiment, the present invention relates to the method as disclosed herein for producing the composition according to the present invention, wherein the protein or protein-type compound as disclosed herein, particularly the enzyme or enzyme-type compound, are covalently bound at the surface of the solid carrier.
The method according to the invention and as described herein may thus comprise the additional step of activating the surface of the carrier material prior to binding the protein or protein-type compound to the surface of the carrier material and in a specific aspect, this may be achieved by homogeneously distributing a linking means on the surface of the carrier material.

In this context, homogeneous distribution of the linking means relates to the linking means being bound on the surface of the carrier material by equal or at least comparable spacing between them. This is achieved in particular where the functional constituents immobilized on the carrier surface will not obstruct or otherwise interfere with each other; preferably, space adequate for binding monomers to the carrier surface is also left. Homogeneity of binding sites presented by linking means may depend on homogeneity of the surface of the carrier material.

Preferably, the linking means is homogeneously distributed on the surface of the carrier material due to the provision of a patterned surface of the carrier material.

The patterned surface may be obtained in various ways such as by preparing a surface being composed of particles, i.e. nanoparticles, wherein each particle has a predefined diameter. Further, a patterned surface may be obtained by structuring the surface with attractant and non-attractant areas being homogenously distributed on the surface of the carrier material. The attractant areas, for example, have an affinity to a linking mean. In contrast, the non-attractant areas have reduced or no affinity to the linking means and, thus, the linking means is not able to bind to the surface of the carrier material. Such structured surfaces may be obtained by well known techniques, e.g. photolithographic approach or microcontact-printing.

So, it is noted that it is possible that only a part of the surface area is modified to improve immobilization of the functional constituent, while other parts remain unmodified and that in such a case the monomers are preferably (also) capable of binding interaction with the unmodified parts of the carrier surface. In this way, the protection layer will be particular tight as it binds to both the functional constituent and the carrier surface. Accordingly, a particularly stable composition can be obtained.

In this respect, it will also be understood by the skilled person that the kind of chemistry applied to the carrier, such as amino modification, and more precisely the density of modifying amino groups, may affect the protein stability and activity at the immobilization stage. As a cross-linker used for immobilization (e.g. glutaraldehyde) is generally binding the protein covalently to the support material, a high density of surface amino groups and therefore a high density of anchoring point would fix, stretch, flat and finally unfold the enzyme, thus most likely effecting the protein structure, which will therefore lose the function. In this context, it is noted that generally the use of the cross-linker such that first particles are modified with the cross-linker and then protein is added will not effect the protein structure or at least effect the protein structure less than a different approach wherein first protein adsorption on particles is effected and then cross linker is added, which is more likely to result in altered protein structure, as the excess of cross linker may "fix" or "stretch" the protein.

Hence, an appropriate density of anchoring amino groups should be reached on the particles surface in order to obtain a stable immobilization. Too few anchoring amino groups will resuit in a leakage of the weakly bound protein. On the contrary, too high anchoring amino groups density may result in an unfolding of the immobilized protein.

As used herein, the term "linking means" relates, e.g., to cross-linking reagents or cross-linkers containing reactive ends, which are capable of binding to specific functional groups (e.g. primary amines, sulfhydryls, etc.) such that one end of the cross-linking reagent or the cross-linker binds to the surface of the carrier material and the other end to a protein or protein-type compound. Cross-linkers may be used to modify nucleic acids, proteins, polymers and solid surfaces or solid templates. For example, a cross-linker may be immobilized on a silica surface as a carrier material and then a protein or protein-type compound may be bound to an unoccupied binding-site of the cross-linker. Alternatively, the cross-linker may firstly bind to the protein or protein-type compound and then the cross-linker bound to the protein or protein-type compound may bind with its unoccupied binding-site to the silica surface. The cross-linker used within the method according to the present invention may depend on the type of carrier material to be used such as inorganic oxides such as silicon oxides or titanium oxides, organic, inorganic, polymeric or inorganic-organic composites and self-assembled organic material. Preferably, the cross-linker may be a cleavable cross-linker, i.e. a cross-linker being capable of having its linkage cleaved upon external stimuli such as temperature, pH, electricity, light. In particular a cross-linker maybe used such as DTSSP (3,3'-Dithiobis[sulfosuccinimidylpropionate]), which can be cleaved, for example, by using DTT (Dithiothreitol) as a reducing agent.

As a non-limiting example, an amino-modified silica surface may be used as a carrier material modified with a homo-bifunctional cross-linker (e.g. glutaraldehyde) forming a Schiff base with the amine group at the surface of the carrier material. The remaining free aldehyde group can then form another Schiff base with the protein or protein-type compound, and, thus, the protein or protein-type compound can be linked to the surface of the carrier material. Care should be taken as the protein or protein-type compound might be released in acidic conditions.

If a gold or titan surface is used as a carrier material, a suitable cross-linker for linking the template to the respective surface may have a thiol end group enabling binding to the respective surface and further a cleavable intramolecular disulfide bond.

It is preferred if the functional constituent, that is the protein or protein-type compound is bound to the surface of the carrier material by covalent binding. As this means that the surface of the carrier is capable of covalent binding, the monomers may be adapted to interact with the modified surface via such binding as well. Generally, it is preferred if the monomers bind directly to this surface, and it is possible to prepare the surface so as to improve immobilization.

Hence, the composition may further comprise at least one bi-functional cross-linker to bind the at least one functional constituent, selected from a protein and a protein-type compound to the surface of the solid carrier, particularly a cross-linker for cross-linking amine to sulfhydryl (thiol) functions and/or a cross-linker for cross-linking sulhydryl to sulfhydryl (thiol) functions, and/or a bi-functional cross-linker selected from the group of glutaraldehyde, disuccinimidyl tartrate, bis[sulfosuccinimidyl] suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, activated sulfhydryls, sulfhydryl-reactive 2-pyridyldithio), BSOCOES (Bis[2-(succinimidooxycarbonyloxy)ethyl]sulfone), DSP (Dithiobis[succinimidyl propionate]), DTSSP (3,3'-Dithiobis[sulfosuccinimidylpropionate]), DTBP (Dimethyl 3,3'-dithiobispropionimidate·2 HCl), DST (Disuccinimidyl tartarate), Sulfo-LC-SMPT (4-Sulfosuccinimidyl-6-[α-methyl-α-(2-pyridyldithio)toluamido]hexanoate), SPDP (N - Succinimidyl 3-(2-pyridyldithio)-propionate), LC-SPDP (Succinimidyl 6-(3-[2-pyridyldithio]-propionamido)hexanoate), SMPT (4-Succinimidyloxycarbonyl-α-methyl-α-[2-pyridyldithio]toluene), DPDPB (1,4-Di-[3'-(2'-pyridyldithio)-propionamido]butane), DTME (Dithio-bismaleimidoethane), BMDB (1,4 bismaleimidyl-2,3-dihydroxybutane).

It is not necessary that the functional constituent is immobilized on the carrier surface in a particular orientation. Rather, a good protective effect can even be obtained according to the invention if the orientation of the molecules of the functional constituent immobilized on the carrier surface is random. This may be due to the fact that for most functional constituents a plurality of interaction sites with monomers exist in every orientation, so that independent of the specific orientation of a given molecule, a tight binding of the protective layer will result.

Also, as long the substrate will be able to penetrate the protective layer and /or to reach the active site of the enzyme or other functional constituent, the orientation of the immobilized enzyme on the surface of the carrier is not vital to be considered. Now, as the protective layer can be designed to be rather thin and thus can be designed to be easily penetrable by the target molecule which is to interact with the immobilized and protected functional constituent, the protective layer will hardly adversely affect the activity regardless of the orientation of the enzyme of functional constituent. Nonetheless, in most cases, the protection layer will be thick enough to retain the functional constituent in a form locking manner, thus providing polymerized e.g. polycondensated material built from the monomers above at least part of the functional constituent molecules such that release is impaired or prevented. With functional constituent having circular molecular cross sections, this is obviously the case where the protection layer is thicker than 50% of the cross section diameter. In such a case, where form locking occurs, the molecules are not only maintained by the weak interaction with the building blocks, but will also be maintained because the building blocks above the functional constituent molecules will be cross-linked with other building blocks forming the polymer and removing the functional constituent molecules would thus require that such cross-linking forces be overcome. This is in stark contrast to a recognition layer formed for releasing e.g. viral particles in virus imprinting technology. The average skilled person will realize that providing pores within the protective layer is particularly effective to allow the use of layers reaching or exceeding 100% of the relevant cross section diameter of a functional constituent even if diffusiuon of target material molecules which are to interact with the functional constituent through such layers is otherwise slow or impossible. Hence, providing pores is helpful in better retaining the functional constituents. Hence, a porous, functional constituent retaining layer is particularly preferred as protective layer.

Accordingly, as it is not necessary to immobilize the molecules of the functional constituent in a particular orientation, it is easier to carry out the method according to the present invention.
However, should orientation be preferred, such orientation can be obtained by applying an enzyme-specific solution for example immobilizing the enzyme in presence of a specific substrate or in presence of solvent, or by using a specific immobilization strategy as known per se in the art.

Also, it is not necessary to add only the functional constituents to the composition. Instead, it is possible that the composition further comprises at least one sort of functional (auxiliary) molecule selected from the groups of adaptor molecules, anchoring molecules, scaffold molecules and/or receptor molecules. The present invention thus further relates in a particular embodiment to a composition of the invention as disclosed herein, wherein said composition optionally further comprises at least one molecule selected from the groups of adaptor molecules, anchoring molecules, scaffold molecules and/or receptor molecules. Any of these molecules can be used to bind, stabilize, capture, trap or catch a substrate (target) molecule. This allows bringing the substrate or interaction partner closer to the functional constituent, that is, the protein or protein-type compound, particularly the enzyme or enzyme-type compound, and to so facilitate interaction of the protein or protein-type compound and its substrate or interaction partner.

Accordingly, using such functional (auxiliary) molecules may help in immobilizing the functional constituent and /or in stabilizing the functional constituent once immobilized or prior to the building of the protective layer. In case such functional (auxiliary) molecules are used, it will be advantageous if the monomer building blocks are selected such as to be further capable of interacting with at least one sort of functional molecules selected from the groups of adaptor molecules, anchoring molecules, scaffold molecules and/or receptor molecules so that the protective layer for protecting the functional constituent is also embedding the at least one sort of functional molecules.

In a particular preferred embodiment the protective layer built according to the invention will be a porous layer.

In this context, it will be obvious to the skilled person that to make use of the functional constituent specific areas thereof must be accessed by the specific molecules or group of molecules the composition of the present invention is to be used with. Now, such "target" molecules may either have direct access to a specific site of the functional constituent in case the functional constituent is only partially embedded and the respective specific area is not covered or, otherwise, access must be effected through the protective layer. In that case, pores need to be provided that give the "target" molecules access to the specific sites of the immobilized and protected functional constituent.

From the above, it is obvious that the present invention may for such a case and a particular non-limiting embodiment be expressed to relate to a method for producing a composition comprising the steps of obtaining a solid carrier; and immobilizing at least one protein or protein-type compound of interest, particularly at least one enzyme or enzyme-type compound, (and, as will be obvious from the present disclosure) optionally, at least one optional molecule at the surface of the carrier; and to then incubate the at least one protein or protein-type compound and, if applicable, the optional molecule bound at the surface of the solid carrier with self-assembling building-blocks to yield a porous nano-environment around the free surface of the solid carrier and the at least one protein and or protein-type compound and optional molecule bound at the surface of the solid carrier.

As has been stated above, the building of the protective layer can be controlled precisely and a layer with specific properties is easily obtained. In particular, it has been stated above that the thickness of the layer may be controlled precisely.

It can be expected that in most cases, protection will be better if the layer is thick enough to warrant sufficient protection against adverse influences while a layer too thick might impair the activity. Such impairing will be caused because where insufficient pores are provided in a layer too thick, access of the target molecules to the immobilized and protected functional constituent is prevented, while in case where particularly large and/or numerous pores are provided, protection might be impaired as the layer will not encapsulate the functional constituent sufficiently. Therefore, it can be expected that an optimum thickness for the protective layer will exist.

It is advantageous if the thickness of the protective layer is at least 5% of the length of the longer axis of the at least one functional constituent, preferably between 50% and 150% of the length of the longer axis of the at least one functional constituent. In a typical case, the thickness of the protective layer ranges from 1 to 100 nm, more typical from 1 nm to 50 nm, more typical from 1 nm to 30 nm, more typical from 1 nm to 25 nm, more typical from 1 nm to 20 nm, more typical from 1 nm to 15 nm, preferably from 5 nm to 15 nm. It will be understood that the optimum thickness of the layer might vary with the specific functional constituent considered, with the specific target molecule and even with the process parameter the composition is likely to be used with. It will also be understood that such optimum thickness can be determined with a simple series of measurements comparing activities obtained with different thicknesses of the protective layer under otherwise identical conditions. Hence, adaption to specific process conditions is possible. Again, it can be noted that a preferred value of the thickness can be determined experimentally. Hence, it will be understood that in certain cases the protective material as provided by the composition of the invention and as disclosed herein, can be adapted to the specific needs and may have a thickness of between 2 nm and 50 nm, particularly between 5 nm and 40 nm, particularly between 5 nm and 25 nm, particularly between 10 nm and 25 nm, particularly between 5 nm and 20 nm, particularly between 10 nm and 20 nm, particularly between 15 nm and 25 nm, particularly between 15 nm and 20 nm, particularly between 20 nm and 25 nm or more than 25 nm.

It should also be noted that in case both a protective layer thickness and a specific pore size need to be determined, this can be done iteratively, e.g. by first determining an optimum layer thickness with a pore size corresponding at least to the size of the target molecule in a first series of experiments, to then readjust the pore size for optimum activity with the layer thickness previously found and, if necessary, readjust the layer thickness for optimum activity with the improved pore size. It will be found that a good activity will be obtained within few iterative steps.
Given the above, it will be obvious that pores in the protective layer will be particularly advantageous if the protective layer is at least 50% of the length of the longer axis of the functional constituent molecule. In this context, it may be assumed that either the immobilization of the functional constituent is effected in a random orientation. If the functional constituent molecules are immobilized in an oriented manner, e.g. with the longer axis generally parallel to the carrier surface, and the aspect ratio of long and short axis of the functional constituent molecules is high, it may be advantageous that pores are provided even if the thickness is lower than 30% of the longer axis. The same holds where a functional constituent molecule has large side arms and the like. Also, even for thin layers pores may be advantageous where target molecules are rather large or bulky and hence access of the target molecules to the functional constituents is impaired.

Thus, it will be understood that on occasions it might be advantageous that the size of the protective material as provided by the composition of the invention and as disclosed herein, is adapted to the specific needs and may be shaped such that about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95% or 100% of the functional constituent, that is, the immobilized protein or protein-type compound, particularly of the immobilized enzyme or enzyme-type compound, is covered by the protective material. Furthermore, it will be understood that providing pores might be advantageous for layer thicknesses of from about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95% or 100% as measured by ratio of the longer axis of the functional constituent molecule and layer thickness. Typically, if the layer is thicker, pores become more and more advantageous.

If pores are provided, it is advantageous if the pore size are between 1 nm and 10 nm, particularly between 2 nm and 9 nm, particularly between 3 nm and 8 nm, particularly between 4 nm and 7 nm, particularly between 4 nm and 6 nm, particularly between 4 nm and 5 nm. From the above, it will be obvious that the actual pore size will depend on the specific target molecule(s) having to reach the functional constituent. It is advantageous if the pore size is dimensioned so as to allow for diffusion of molecules to the functional constituent for interaction therewith during use of the composition. Hence, the pores will have a size corresponding at least to the size of the target molecule. It will be understood by the person skilled in the art that the different pore sizes indicated to be preferred will relate to different specific target molecules and/or to layers of different thickness; for a thicker layer, a larger pore size may be advantageous given that the larger pores will allow percolation of target molecules to the functional site(s) of the functional constituent more easily and/or to a larger degree.

In this respect it should be noted that the pore size can be influenced and adjusted. Therefore, the size of the pores present in the protective material provided by the composition of the invention and as disclosed herein, can be adapted to the specific needs, wherein said pore size is chosen such that it allows the diffusion of molecules, which are to interact with the protein or protein-type compounds forming said functional constituent. The pore size can be regulated by the selection of particular building blocks having desired functional groups. Generally, the use of larger functional groups results in increased porosity compared to the use of smaller functional groups. For example, monomers as building blocks for the protective layer can be used at least part of which have three chemical groups that form covalent bonds and a fourth group that interacts with the functional constituent in a non-covalent manner. Also, a surfactant might be introduced at the critical micelle concentration during the protective layer formation. Then, monomers can be added that carry large and bulky groups. As an example, as octadecylsilane and triphenylysilane e.g. octadecyltrimethoxysilane and triphenyl-triethoxysilane may be used used where organo silanes are the building block monomers.

The person skilled in the art will understand that it is even possible to vary the pore size along the way from the surface of the protective layer to the carrier surface. Such variation is feasible because the built-up of the protective layer is slow enough to vary during the built-up process the process conditions such that different pore sizes are obtained, e.g. by adding certain components at a later stage only.

In a particularly preferred embodiment, the protective material may be further chemically modified at its outer surface in order to introduce additional functionalities, particularly by improving the affinity of the produced protective layer for molecules, which are to interact with the protein or protein-type compounds such as, for example, a substrate of an enzyme, in order to create a gradient at the surface of the protective layer.

It is advantageous if the monomers capable of interacting with each other and the immobilized functional constituent are provided as an aqueous solution. Using an aqueous solution of monomers helps preventing damage from the conformation of the functional constituent and thus maintain activity thereof.

It is advantageous if the building blocks build the protective layer in a self-assembling reaction. While polymerization can be e.g. a radical polymerization using a surface bound or soluble initiator, water-soluble unsaturated monomers or a water soluble cross-linker, for the purpose of the present invention, the polymerization reaction is frequently a polycondensation, typically of silica precursors such as tri-alkoxy-silane and tetra-alkoxy-silane under aqueous conditions. Using a polycondensation reaction is therefore a preferred self-assembling reaction.

In most cases it will be preferred to not add certain chemicals that are otherwise used in polymerization such as starters and the like which might impair the activity of the yet-unprotected proteins or protein-type compounds. It is noted that polycondensation can be effected in aqueous solutions and that doing so is particularly preferred as this generally warrants a bio-friendly reaction environment so that in this way, conformation of the functional constituent is preserved or at least adversely affected to only a small degree so that activity thereof is impaired only to a small degree as well if any. Also, polycondensation can be easily controlled, allowing that thicknesses of the protective layer are achieved that are compatible with enzyme protection and substrate diffusion (such as enzyme larger axis where the functional constituent is an enzyme, or 50% more), resulting in a active protected enzyme. It is noted that organo silane monomers may advantageously be used in this context.

It is advantageous if the method for producing a composition according to one of the previous claims further comprises the step of stopping the protective layer building and/or self-assembly reaction of the protective material to so obtain a preferred protective layer with a desired thickness. Stopping the polymerization of the protective material on the surface of the carrier material (and/or on the protective embedding layer as far as such layer has been built up already) can be performed by actively stopping the polymerization reaction or by self-stopping of the polymerization reaction.

It is advantageous if the interaction between the monomer building blocks for the protective layer and the immobilized functional constituent is effected between amino acid side chains of the protein or protein-type compound of the functional constituent, particularly based on weak force interactions.

Such amino acid side chains will be present at the surface of the molecules of the functional constituent to be embedded according to the present invention. As different acid side chains will interact via different weak force interactions, it is also advantageous if a plurality of different building blocks are provided such that the different building blocks interact with different functional parts and/or different amino acid side chains. In this context, weak force interaction relate to non-covalent binding and/or p-p (aromatic) interactions, van der Waals interactions, H-bonding interactions, ionic interactions. From the fact that while the interaction of monomers with each other can will preferably be a polymerization reaction whereas the interaction of the monomers with the functional constituent is preferably a weak interaction, it can be deduced that the monomer-monomer-interaction need not be the same interaction as the interaction of the monomers with the functional constituent. Instead, typically the interaction will be a different one. Furthermore, binding interaction of the monomers to the surface of the solid carrier may by a covalent binding.

If monomers are used having at least one functional group for interacting with the immobilized functional constituent it is thus preferable if these are selected from an alcohol, an amine, a carboxylate, an aromatic function, a thiol, a thioether, a guanidinium, an imidazole, an aliphatic chain, an amide and/or a phenol, so that the functional group may interact with one or more amino acid side chains of amino acids residing on the surface of the protein or protein-type compound by weak force interactions.

It should be noted that while a plurality of different monomers having such functional groups are available, the use of organosilanes is usually particularly preferred.

In a specific embodiment, the present invention thus relates to the composition as disclosed herein, wherein the protective material is organosilica.

Hence, in a particularly preferred embodiment, organo silane monomers are used as building blocks for building the protective layer at least partially embedding the functional constituent. In that case, it is advantageous if organo silane monomers are used having at least one functional group for interacting with the immobilized functional constituent selected from an alcohol, an amine, a carboxylate, an aromatic function, a thiol, a thioether, a guanidinium, an imidazole, an aliphatic chain, an amide and/or a phenol, in particular a functional group which interacts with one or more amino acid side chains of amino acids residing on the surface of the protein or protein-type compound by weak force interactions. In this context, weak force interaction relate to non-covalent binding and/or p-p (aromatic) interactions, van der Waals interactions, H-bonding interactions and ionic (electrostatic) interactions.

The organo silane monomers will preferably be selected from the group consisting of tetraorthosilicate, carboxyethylsilanetriol. and/or benzylsilanes, propylsilanes, isobutylsilanes, n-octylsilanes, hydroxysilanes, bis(2-hydroxyethyl)-3-aminopropylsilanes, aminopropylsilanes, Ureidopropylsilanes, (N-Acetylglycyl)-3-aminopropylsilanes in particular selected from benzyltriethoxysilane, propyltriethoxysilane, isobutyltriethoxysilane, n-octyltriethoxysilane, hydroxymethyltriethoxysilane, bis(2-hydroxyethyl)-3-aminopropyltriethoxysilane, aminopropyltriethoxysilane, Ureidopropyltriethoxysilane (N-Acetylglycyl)-3-aminopropyltriethoxysilane and/or selected from benzyltrimethoxysilane, propyltrimethoxysilane, isobutyltrimethoxysilane, n-octyltrimethoxysilane, hydroxymethyltrimethoxysilane, bis(2-hydroxyethyl)-3 -aminopropyltrimethoxysilane, aminopropyltrimethoxysilane, Ureidopropyltrimethoxysilane (N-Acetylglycyl)-3-aminopropyltrimethoxysilane and/or selected from benzyltrihydroxyethoxysilane, propyltrihydroxyethoxysilane, isobutyltrihydroxyethoxysilane, n-octyltrihydroxyethoxysilane, hydroxymethyltrihydroxyethoxysilane, bis(2-hydroxyethyl)-3 - aminopropyltrihydroxyethoxysilane, aminopropyltrihydroxyethoxysilane, Ureidopropyltri-hydroxyethoxysilane, (N-Acetylglycyl)-3-aminopropyltrihydroxymethoxysilane. It should be noted that these silanes are preferred as

they are commercially available. It will hence be obvious to the skilled person in view of the present disclosure that other organo silanes available at present or in the future might be used as well and that hence while indicating preferred monomers, the list is not excluding other monomers from the disclosure.

As different surface amino side chains will generally be present at the surface of a functional constituent, in a particularly preferred embodiment different organo silane monomers are used instead of one single monomer. The average skilled person will be aware that the different surface amino acids will interact via different interaction (binding) mechanisms. This also holds for monomers having different functional groups and it is preferred to use a mixture of monomers optimized with respect to the amounts of different surface amino acids.

Preferably, the method according to the present invention therefore comprises the steps of analysing or determining a surface structure of the protein or protein-type compound prior to providing the building blocks, and choosing the building blocks corresponding to the surface structure. This step can be useful for enabling a specific binding of the protein or protein-type compound to the protective material, particularly, if the protein or protein-type compound has a known structure as mentioned above.

If the protein or protein-type compound has a known structure, chemical functions on the surface of the protein or protein-type compound can be identified. Thus, selection of building blocks used to prepare the protective material may be dependent on the known structure of the protein or protein-type compound in order to adapt the affinity of the protective material. The choice of the building blocks, which can be used to prepare the protective material, may depend on the known structure of the protein or protein-type compound in order to adapt the affinity of the protective material to its respective need. The composition of the protective material depends on the compounds present in the reaction mixtures such as structural building blocks (e.g. tetraethylorthosilicate (TEOS)) and/or protective building blocks (e.g. tetraethylorthosilicate (TEOS), 3-Aminopropyltriethoxysilane (APTES), n-Propyltriethyoxysilane (PTES), Isobutyltriethoxysilane (IBTES), Hydroxymethyltriethoxysilane (HTMEOS), Benzyltriethoxysilane (BTES), Ureidopropyltriethoxysilane (UPTES), Carboxyethyltriethoxysilane (CETES)) and a self pre-organizing of these building blocks around the protein or protein-type compound via weak force interactions such as hydrogen bonding, electrostatic interactions, hydrophobic interactions or van-der-Waals interactions, π - π stacking [(pi-pi) stacking].

Accordingly, it is advantageous if for at least one functional constituent, the respective amount of at least several of the surface amino acids selected from the group consisting of Phe, Tyr, Trp, Gly, Ala, Leu, Ile, Val, Pro, Ser, Thr, Asp, Asn, Gln, Asp, Glu, Lys, Arg, His is determined and different monomers are used in accordance with the determination.

Therefore, it is advantageous if the amount of at least one of Phe, Tyr, Trp as surface amino acids of the at least one functional constituent is determined. Such determination may either relate to the amount of only one of said surface amino acids or to the amount of several of said surface amino acids, preferably the sum of all of them. Then an amount of monomer(s) having a functional group interacting with the surface amino acids Phe, Tyr, Trp of the functional constituent through p-p (aromatic) interactions is selected according to this determination, in particular an amount of benzylsilanes, in particular one or more of a benzyltriethoxysilane, benzyltrimethoxysilane or benzyltrihydroxyethoxysilane. It is noted that the determination may either relate to the amount of only one of said surface amino acids or to the amount of several of said surface amino acids, preferably (the sum of) all of them. Then, when the amount of monomer(s) having a functional group interacting with the mentioned surface amino acids of the functional constituent is set according to this determination, one or more monomers having the respective interaction can be selected for building the protective layer.

In addition and/or as an alternative the amount of at least one Gly, Ala, Leu, Ile, Val, Pro as surface amino acids of the at least one functional constituent is determined and an amount of monomer having a functional group interacting with the surface amino acids Gly, Ala, Leu, Ile, Val, Pro of the functional constituent through van der Waals interactions is selected according to the determination, in particular an amount of at least one of propylsilanes, isobutylsilanes, n-octylsilanes in particular one of a propyltrimethoxysilane, isobutyltriethoxysilane, or a n-octyltriethoxysilane and/or one of propyltriethoxysilane, isobutyltriethoxysilane, n-octyltriethoxysilane, and/or propyltrihydroxyethoxysilane, isobutyltrihydroxyethoxysilane, n-octyltrihydroxyethoxysilane. Again, the determination may either relate to the amount of only one of said surface amino acids or to the amount of several of said surface amino acids, preferably the sum of all of them. Then, when the amount of monomer(s) having a functional group interacting with the mentioned surface amino acids of the functional constituent is set according to this determination, one or more monomers having the respective interaction can be selected for building the protective layer.

In addition and/or as an alternative the amount of at least one of Ser, Thr, Asp, Glu, Asn, Gln, Tyr as surface amino acids of the at least one functional constituent is determined and an amount of monomer having a functional group interacting with the surface amino acids Ser, Thr, Asp, Glu, Asn, Gln, Tyr of the functional constituent through H-bonding interactions is selected according to the determination, in particular an amount of at least one of hydroxysilanes, bis(2-hydroxyethyl)-3-aminopropylsilanes, in particular one of hydroxymethyltriethoxysilane, bis(2-hydroxyethyl)-3-aminopropyltriethoxysilane, and/or one of hydroxymethyltrimethoxysilane, bis(2-hydroxyethyl)-3-aminopropyltrimethoxysilane and/or one of hydroxymethyltrihydroxyethoxysilane, bis(2-hydroxyethyl)-3-aminopropyltrihydroxyethoxysilane. Again, when the amount of monomer(s) having a functional group interacting with the mentioned surface amino acids of the functional constituent is set according to this determination, one or more monomers having the respective interaction can be selected for building the protective layer.

In addition and/or as an alternative the amount of at least one of Asp, Glu as surface amino acids of the at least one functional constituent is determined and an amount of monomer having a functional group interacting with the surface amino acids Asp, Glu of the functional constituent through ionic interactions is selected according to the determination, in particular an amount of aminopropylsilanes, in particular at least one of aminopropyltrimethoxysilane, aminopropyltrihydroxyethoxysilane aminopropyltriethoxysilane. Again, when the amount of monomer(s) having a functional group interacting with the mentioned surface amino acids of the functional constituent is set according to this determination, one or more monomers having the respective interaction can be selected for building the protective layer.

It is noted that some surface amino acid side chains may interact via different mechanisms. Where monomer mixtures are adapted according to the amount of specific surface amino acid side chains, this can be taken into account.

What follows from the above is, that e.g. when based on an amino acid surface analysis of the target enzyme, the majority of amino acid present at the surface of the enzyme defining the functional constituent are negatively charged, then more organosilanes having positively charged functional groups should be added in the building blocks mixture. In this way, the organosilanes of the mixture will better interact (in a non-covalent manner) with the enzyme to be protected by embedding for use.

While the carrier can be any solid carrier, e.g. a chip or the like, it is advantageous in most applications if the solid carrier is a particulate carrier, in particular with a particle size in a range of between 20 and 1000 nm, particularly of between 200 and 500 nm, particularly between 300 and 400 nm.

In this context, it should be noted that the number of proteins or protein-type compound molecules, that is, the number of individual functional constituent molecules that can or will be bound to a given particle will depend on the ratio of the size of the carrier particle to the size of said protein or protein-type compound molecule. For larger particles, there may obviously be statistic variations of said number. If the protein or protein-type compound has a similar size as the nanoparticle, one protein or protein-type compound molecule may be bound per particle. A similar size refers in this instance to a difference in size which is in the range of between 0,5% and 10%.

In one embodiment, the size ratio of particle to protein or protein-type compound is such that it allows binding of between 10 to 200, particularly 50-250, particularly 20-150 proteins or protein-type compounds per nanoparticle. In a specific embodiment, the nanoparticle has a size which allows binding of 200 proteins or protein-type compounds per nanoparticle. Such a ratio is preferred because with such a ratio, adding a small amount of composition into a process vessel, organism or the like will equal addition of a rather large amount of active functional constituents.

It should however be noted that using a rather large particulate carrier allows to separate the composition from a fluid by filtering after use. While in some instances, it might be more preferred to use a small particle carrier, e.g. to allow transport of the composition within a living organism, there might also be occasions where larger sized particles will be used within the scope of the present invention, particularly particles with a size of at least 1000 nm and up to 100 µm.

The carrier may hence be a nanoparticle, particularly a nanoparticle selected from the group consisting of organic nanoparticle, inorganic nanoparticle, organic-inorganic composite nanoparticle, self-assembling organic nanoparticle, mesoporous silica nanoparticle (SNP), gold nanoparticle and titanium nanoparticle.

Irrespective of the size of the carrier, the carrier material may have a silicium oxide surface which is particularly preferred where organo silanes are used as monomers.

It is possible and advantageous if said functional constituent selected from a protein and a protein-type compound is an enzyme or enzyme-type compound, particularly an enzyme or enzyme-type compound, which is selected from the group consisting of oxidoreductases, transferases, hydrolases, lyases, isomerases and/or ligases.

Protection is also sought for a composition comprising a solid carrier, at least one functional constituent, selected from a protein and a protein-type compound , and immobilized on the surface of the solid carrier, and a protective layer for protecting the functional constituent by at least partially embedding the functional constituent, wherein the protective layer for protecting the functional constituent is a layer built with building blocks monomers which are capable of interacting with each other and the immobilized functional constituent.

Again, embedding will be such that (at least a majority or large fraction of) the functional constituent will remain embedded until use, preferably until end of use, and is neither intended to be washed out nor will washing out take place during intended use.

The average skilled reader is aware that proteins comprise an extremely heterogeneous class of biological macromolecules. Many are unstable when not in their native environments. If certain buffer conditions are not maintained, extracted proteins may not function properly or remain soluble. Proteins can lose structural integrity and activity as a result of suboptimal temperature, proteolysis, aggregation and suboptimal buffer conditions.

According to the present invention the protective material of the composition as disclosed herein provides protection for the protein or protein-type compound, particularly the enzyme or enzyme-type compound to environmental conditions, which deviate from the native conditions.
In particular, the protein or protein-type compound, particularly the enzyme or enzyme-type compound is protected against:
a) a suboptimal pH; and/or
b) chemical stresses; and/or
c) biological stresses; and/or
d) solvents; and/or
e) physical stress
A "suboptimal pH" refers to a pH, which differs from the optimal pH for the at least one protein or protein-type compound by a value of e.g. +/- 5, +/- 4; +/- 3, +/- 2, +/- 1, +/- 0.5 pH units; it will be understood by the average skilled person that where the composition of the invention will be subject to more extreme conditions, the protective layer may be adapted to such conditions.
The term "chemical stress" as used herein comprises, but is not limited to, conditions caused by dilution in solvent, pollution with unwanted reactants, e.g. pesticides, insecticides, polluted air and/or water, heavy metals such as mercury or lead, asbestos or radioactive waste, compounds used in chemotherapy, or toxins. Especially enzymes are unstable in solvents that are different from their optimal buffer system.
The term "biological stress" as used herein comprises, but is not limited to, conditions caused by protease activity, oxidative stress, caspase activity, natural enzyme inhibitors, low substrate concentration, bright light exposure, UV light exposure, low ATP levels, contamination with unwanted proteins, phosphatase activity and drug metabolism.
The term "physical stress" as used herein comprises, but is not limited to, shear forces, dryness, pressure and vacuum induced stress.

In one embodiment the present invention relates to the composition as disclosed herein, wherein the protective material provides protection for the protein or protein-type compound against suboptimal temperatures, which may lead to inactivation and/or denaturation of the unprotected protein.

If the protein is an enzyme, the immobilized and protected enzyme as disclosed herein can retain its activity and structural integrity under suboptimal temperatures, which are higher than the optimal reaction temperature and where the enzyme of interest may have reduced activity. In particular, the invention provides for a composition as disclosed herein, wherein the protective material provides protection for the protein or protein-type compound against elevated temperatures, which exceed the optimal temperature for the unprotected protein or protein-type compound by 60°C, particularly by 50°C, particularly by 40°C higher, particularly by 30°C, particularly by 20°C, particularly by 10°C, particularly by 5°C In a specific embodiment, the protein or protein-type compound is an enzyme or enzyme-type compound. The tight encapsulation achieved according to the invention thus protects the functional constituent even under extremely adverse conditions.

It is thus possible and advantageous if the composition is used in a catalytic and/or other industrial processes; the composition can be used in various catalytic processes.

In particular, it is possible to use the composition of the invention in a catalytic process wherein during the process the composition is subject to at least one of a pH different from the optimal pH of the functional constituent in particular such that the pH value differs at least by +/- 0.5 pH units and/or up to +/- 5 pH units from the pH optimal for the functional constituent and/or to chemical stresses; and/or to biological stresses; and/or to solvents; and/or to physical stress; and/or to elevated temperatures, which exceed the optimal temperature for the functional constituent by at least 5°C; and /or up to 60°C, particularly by 50°C, particularly by 40°C higher, particularly by 30°C, particularly by 20°C, particularly by 10°C; and/or to reduced temperatures, which deviate from the optimal temperature for the functional constituent by at least 5°C; and /or up to 60°C.

In one embodiment, the present invention relates to the composition as disclosed herein, wherein the protective material provides protection for the protein or protein-type compound against reduced temperatures, which are lower than the optimal temperature and which may lead to inactivation and/or denaturation of the unprotected protein.

If the protein is an enzyme, the immobilized and protected enzyme as disclosed herein, can retain its activity and structural integrity under suboptimal temperatures, which are lower than the optimal reaction temperature and where the enzyme of interest may have reduced activity. In particular, the invention provides for a composition as disclosed herein, wherein the protective material provides protection for the protein or protein-type compound against reduced temperatures, which deviate from the optimal temperature for the unprotected protein or protein-type compound by 60°C, particularly by 50°C, particularly by 40°C, particularly by 30°C, particularly by 20°C, particularly by 10°C, particularly by 5°C. In a specific embodiment, the protein or protein-type compound is an enzyme or enzyme-type compound.

In another embodiment of the present invention, the composition as disclosed herein is a biocatalytic system. In particular, the at least one protein or protein-type compound of interest is an enzyme or enzyme-type compound.

In particular, the enzyme or enzyme-type compound is selected from the group of oxidoreductases, transferases, hydrolases, lyases, isomerases and/or ligases such as, for example, a laccase, a peroxidase, a phosphatase, a oxygenase, a reductase, a protease, an amylase and/or an esterase.

Hence, in particular, the composition according to the present invention and as disclosed herein can be used in food processing or brewing. Thus, the composition according to the present invention and as disclosed herein may be used in food processing, particularly for processing of dairy products, brewing, for processing of fruit juice, particularly fruit juice clearance, sugar production, tendered meat production, wine production, etc. Further, the composition according to the present invention and as disclosed herein may be used in decontamination processes, detoxification processes, in starch industry, paper industry, biofuel industry, rubber industry, photographic industry, or in detergent production. In particular, the disclosed composition according to the present invention can be recycled for being reused in additional reaction cycles. Here, the use of the composition according to the invention for catalytic processes is highly advantageous.

Further, the composition of the present invention can also be used in decontamination processes, particularly enzyme-dependent decontamination processes. For example, the composition can be used to remove or degrade unwanted chemical compounds, such as hydrocarbons, aromatic hydrocarbons, pesticides, toxins, solvents, agricultural chemicals and/or heavy metals. The composition of the present invention thus can be used to clean contaminated soil or water. For example, the composition according to the present invention can thus be used to purify wastewater by removing or degrading a contaminant.

The composition according to the present invention can be directly used for the preparation of packed-bed reactor systems to treat water via a percolation process. A further possible technology is the immobilization/embedment of proteins or protein-type compounds in filtration membranes for treating effluents. This application may be seen in the format of a filter for the depuration of domestic water, which may be relevant in developing countries.

In principal, the composition according to the present invention can be used in any catalytical process that is, in any process which is based on the use of at least one particular protein, particularly an enzyme.

The use of the composition according to the present invention instead of the native protein or enzyme has the advantage that the protein or enzyme can be recycled and, thus, be used again in another reaction cycle. This reduces the cost and effort for producing or isolating the protein of interest, particularly, the enzyme of interest. If the composition is used instead of the unprotected protein of interest, the required total amount of protein of interest is less, as more than a single biocatalytical reaction can be achieved. Further, the composition of the present invention can be produced in large scale, which makes it especially suitable for the use in industrial manufacturing processes, where a high through-put of substrate is often required. Isolated or recombinant proteins, especially enzymes have found several applications within a broad variety of industrial branches. Enzymes are for example used in food industry, chemical industry and protein engineering. In particular, they can be used for the production of enantiomerically pure amino acids, rare sugars, such as fructose, penicillin and derivatives thereof, washing agents, as well as other chemical compounds. The immobilized and protected enzyme according to the present invention may also be genetically optimized to increase its biocatalytical activity.

The fact that the composition of the present invention provides for a particularly good protection of the functional constituent thus allows the use of the composition under extremely adverse conditions. This is advantageous as it frequently opens the path to entirely new applications or to the use of material otherwise too expensive to use due to fast decrease of activity and the thus resulting necessity to increase the amount of (unprotected) functional constituents.

The present invention further relates to the composition as disclosed herein, wherein the composition is non-toxic. Further, the composition according to the present invention is amenable to large-scale production.

It is therefore possible and advantageous if the composition is used in therapy of human or animal, particularly mammal therapy. The composition of the present invention as described herein can therefore be formulated as a pharmaceutical composition, so that it may be used in human or animal therapy.

In particular, the composition may be used in therapy of one of sphingomyelinase deficiency (ASMD) syndrome, Niemann-Pick Disease (NPD), lysosomal storage diseases, Gaucher disease, Fabry disease, mucopolysaccharidosis (MPS) I, MPS II, MPS VI and Glycogen storage disease type II, cancer, allergic diseases, metabolic diseases, cardiovascular diseases, autoimmune diseases, nervous system disease, lymphatic disease and viral disease.
The composition according to the invention as described herein may be used in enzyme replacement therapy (ERT) in patients suffering from a disease, which is induced by the deficiency or absence of a particular enzyme. The composition comprising the deficient or missing enzyme in the immobilized and protected format of the invention may be administered alone or in combination with other drugs for the use in therapy of said disease in animals, particularly in mammals, more particularly in humans.

In particular, in cancer therapy the composition according to the invention and as described herein may provide an enzyme protected by a protective layer, the catalytic activity of which leads to a depletion of one or more metabolites, which are needed by the cancer cell for survival.

For example, in certain cancers, the cancer cells lack an enzyme, e.g. argininosuccinate synthetase, that makes these cells auxotrophic for arginine. Depleting the level of arginine by providing a composition according to the present invention and as described herein comprising an arginine degrading enzyme such as, for example, an arginase protected by a protective layer, would be fatal for the cancer cells while normal cells remain unaffected or at least are not fatally affected.
In another aspect, the composition according to the present invention and as disclosed herein, may be used for the preparation of a pharmaceutical composition for treating a disease, disorder or condition or symptoms of said disease.
The pharmaceutical composition may comprise in addition to the composition of the invention and as described herein a pharmaceutically acceptable carrier and/or excipient. The composition of the invention may be provided in a therapeutically and/or prophylactically effective amount.
For example, the composition of the invention and as disclosed herein may be formulated as a cream, a tablet, pill, bioadhesive patch, sponge, film, lozenge, hard candy, wafer, sphere, lollipop, disc-shaped structure, or spray.
The composition may be administered to a subject in need thereof by systemic, intranasal, buccal, oral, transmucosal, intratracheal, intravenous, subcutaneous, intraurinary tract, intravaginal, sublingual, intrabronchial, intrapulmonary, transdermal or intramuscular administration.

The composition according to the present invention can be further used to prevent degradation of a protein or protein-type compound, particularly proteasomal degradation of a protein or protein-type compound after administration into the body. Proteins are tagged for proteasomal degradation with a small protein, so-called ubiquitin. The use of the composition according to the invention may prevent ubiquitin-tagging of the protein or protein-type compound of interest and thus also prevents degradation of the protein or protein-type compound by proteasomes. Accordingly, the levels of the administered protein or protein-type compound can be maintained stable over a longer period in the blood and tissue of the patient.

From the above, it will be understood that part of the invention can be considered to inter alia provide a composition comprising at least one protein or protein-type compound immobilized at the surface of a carrier, particularly a solid carrier, wherein said protein or protein-type compound is fully or partially embedded in a protective material comprised of self-assembling building blocks. These building blocks of the protective material can undergo a self-assembly reaction on the carrier surface and around the protein or protein-type compound immobilized on the carrier surface to establish a porous nano-environment around the immobilized protein or protein-type compound. The establishment of said porous nano-environment may be accomplished by the presence of selected functional groups, which are provided by the protective material and which groups interact with the chemical groups of the protein or protein-type compound such that the native conformation of the protein or protein-type compound is stabilized and its function preserved.

According to an even more detailed and specific embodiment, the present invention can be understood to provide inter alia a composition comprising at least one protein or protein-type compound immobilized at the surface of a carrier, particularly a solid carrier, wherein said protein or protein-type compound is fully or partially embedded in a protective material comprised of self-assembling building blocks.

As used herein, protective material can be construed to relate to a material being capable of a polymerization reaction, which material can be provided to the surface of the carrier material. Preferably, the protective material is a monomeric material or contains such monomeric material to a large fraction and is provided in liquid phase. During polymerization, the protective material can self-assemble on the carrier surface and around the at least one protein or protein-type compound immobilized at the surface of the solid carrier. In this way, the at least one protein or protein-type compound becomes embedded in a protective layer grown from the surface of the carrier material into direction of the at least one protein or protein-type compound or from the at least one protein or protein-type compound into direction of the surface of the carrier material or both. After being polymerized, the protective material usually is in solid phase.

Applying a preferred method according to the invention, the polymerized protective material provides a porous nano-environment around the protein or protein-type compound, which is then fully or partially embedded by the protective material. In a preferred embodiment, the protective material will be organosilanes monomer(s).

In a preferred embodiment, the composition is obtained by enzyme immobilization on silica effected by APTES modification of silica and glutaraldehyde crosslinking chemistry, then the self-assembly of organosilanes monomers around the enzyme as a template is effected and a controlled polycondensation of organosilanes monomers in water is effected with the need to use additives during the polycondensation reaction.

Where the expression "fully embedded protein or protein-type compound" is used herein it shall mean that the protein or protein-type compound of interest according to the invention is 100% covered by the self-assembled protective material as defined in various embodiments of the present inventions.

The expression "partially embedded protein or protein-type compound" and similar expressions as used herein shall mean that the protein or protein-type compound according to the invention is not fully covered by the self-assembled protective material as defined in various embodiments of the present inventions, thus, the protein or protein-type compound is not fully embedded in the protective material. In various embodiments of the invention, it is possible that not less than 5% of the protein or protein-type compound of interest are covered by the protective material, though typically more at least 10% will be covered, thus improving protection of the functional constituent. While such a minimum embedding may sometimes be sufficient, in general, an even higher degree of embedding is preferred for better protection and retention of the functional constituent, and hence typically at least 50% embedding will be provided. In a particularly preferred embodiment, the embedding will even be at least 80%, particularly at least 90%, particularly 99% of the protein or protein-type compound of interest are covered by the self-assembled protective material. Hence, it is obvious that it is highly preferred to choose such a degree of embedding that the functional constituent will generally not be washed out. Assuming a circular or cylindrical shape of the molecules of the functional constituent, it will be obvious that while an embedding of at least 50% will be used, typically at least 70% embedding are preferred from mere geometrical considerations, but depending on the presence of side chains and the like, a lesser degree e.g. 30% might already suffice to prevent washing out. Again, it should be noted that a measure of washing out can easily be determined and that accordingly a sufficient thickness of the protective layer or degree of embedding can be determined.

On the other hand, it is not necessary that the thickness of the functional constituent retaining protection layer becomes excessive in order to improve protection. Both retention of the functional molecules by cross-linked material form-fittingly overlapping the functional constituent molecules to a high degree and the protection against attack by chemicals will be sufficient if the functional constituent molecules are almost covered or completely covered (that is to 100%) without the need to further increase the thickness of the protection layer. While some additional protection against chemical attack might be obtained if diffusion is reduced by a thicker layer, there hardly is any need to have a layer thicker than about 200%. Typically the layer will be smaller than 150% of the length of the longer axis of the functional constituent and most frequently the layer will be smaller than about 120%, in preferred cases not even exceeding 110%. Even for small functional constituent molecules the retaining forces from cross-linked material above the layer will be sufficient in most cases and even in abrasive conditions and the like, the endurance of such a layer will be sufficient for most processes.

It has also been already disclosed above that in a specific embodiment of the invention, the protein or protein-type compound is bound to the surface of the carrier material by covalent binding. A covalently bound protein or protein-type compound contributes to a stable surface of the carrier material bearing the protein or protein-type compound which may provide stable conditions for polymerization of the protective material.

It will also be understood by the average skilled person that in a specific embodiment, the protective material as provided in the composition of the present invention and as disclosed herein may be composed of monomeric building blocks, which self-assemble on the free surface of the carrier and around the immobilized protein or protein-type compound immobilized at the surface of the carrier. The monomeric building blocks of the protective material then self-assemble on the free surface of the carrier and form bonds, particularly covalent bonds, with reactive groups provided on the carrier surface and by the self-assembling building blocks, such that a protective layer is generated, which is fixed (by rather strong binding forces) to the carrier surface and provides a porous nano-environment around the immobilized protein or protein-type compound. The porous nano-environment protects the at least one protein or protein-type compound immobilized at the surface of the solid carrier against various stresses including environmental stress, pH stress, biological stress, mechanical stress and/or physical stress as defined herein. The porous nano-environment further allows small molecules to move through the pores and to interact with the immobilized protein or protein-type compound of interest. It will be understood by the average skilled person that while providing pores in the protective layer might reduce the retention force provided by the cross-linked material, such forces will still be more than adequate.

In a particularly preferred embodiment, the protective material may be further chemically modified at its outer surface in order to introduce additional functionalities, particularly by improving the affinity of the produced protective layer for molecules, which are to interact with the protein or protein-type compounds such as, for example, a substrate of an enzyme, in order to create a gradient at the surface of the protective layer.

According to the invention as disclosed herein, it is possible to protect the catalytic site of an embedded, retained enzyme or enzyme-type compound used as a functional constituent and to protect and preserve its functionality. Hence, said immobilized and protected enzyme or enzyme-type compound as provided in the composition of the invention and as disclosed herein has, for example:
a) an increased activity under stress conditions when compared to the free-unprotected enzyme; and/or
b) an increased recoverability for use in continuous operation when compared to the free-unprotected enzyme.

An "increased activity" as used herein is understood to refer to an activity of the immobilized and protected enzyme or enzyme-type compound, which is higher than the activity of the same enzyme or enzyme-type compound when provided in an unbound and non-protected format, when tested in the same test system and under identical conditions. In particular, said increase is about 5%, particularly about 10%, particularly about 15%, particularly about 20%, particularly about 25%; particularly about 30%, particularly about 35%, particularly about 40%, particularly about 45%, particularly about 50%, particularly about 55%, about 60%, particularly about 70%, particularly about 80%, particularly about 90%, particularly about 100%, particularly about 110%, particularly about 120%, particularly about 130%, particularly about 140%, particularly about 150%, particularly about 160%, particularly about 165%. It will be understood that the exact amount of increase of activity will vary with functional constituent, parameters such as pore size necessary, layer thickness and process parameters but that generally, an increase of at least 10% can be expected.

Then, an "increased recoverability" is understood for the purpose of the present invention to refer to the capability of the composition according to the present invention of being reused several times in industrial or laboratory use, that is, where the composition is not administered for therapeutic purposes. In particular, the composition of the invention and as described herein may be reused between 2 and 30 times, particularly between 5 and 30 times, particularly between 10 and 30 times, particularly between 15 and 30 times, particularly between 20 and 30 times, particularly between 25 and 30 times, particularly at least 30 times. Here, "increased recoverability" will depend on both the mechanical recovery of the composition and the prevention of damage to the composition after the actual process, but can generally be expected to be at least be 2 times without significant loss if adequate adaption to an industrial process is observed.

The present invention further relates in a particular embodiment to a composition of the invention as disclosed herein, wherein said composition optionally further comprises at least one molecule selected from the groups of adaptor molecules, anchoring molecules, scaffold molecules and/or receptor molecules. Any of these molecules can be used to bind, stabilize, capture, trap or catch a substrate (target) molecule. This allows bringing the substrate or interaction partner closer to the functional constituent, that is, the protein or protein-type compound, particularly the enzyme or enzyme-type compound, and to so facilitate interaction of the protein or protein-type compound and its substrate or interaction partner.

In one embodiment the present invention relates to the composition as disclosed herein, wherein the protein or protein-type compound and/or at least one of the optional molecules is covalently bound at the surface of the solid carrier. The protein or protein-type compound and/or at least one of the optional molecules can be bound to the surface of the solid carrier by a reactive group, provided on the surface of the carrier such as, for example, a bi-functional cross-linker, particularly glutaraldehyde.

The composition according to the present invention can be used for the diagnosis of a disease or disorder in a sample of a subject to be tested, wherein the protein or protein-type compound immobilized on the surface of the carrier is a capturing molecule, which binds to a specific interaction partner, wherein the presence or absence of said specific interaction partner indicates whether said subject suffers from the disease. An example for a suitable capturing molecule is a specific antibody or functionally equivalent parts thereof.

Further, the composition according to the present invention can be used for the diagnosis of a disease or disorder in a sample of a subject to be tested, wherein the protein or protein-type compound immobilized on the surface of the carrier is an enzyme, wherein the enzyme catalyzes a reaction, which if it takes place indicates the presence of a particular molecule in said sample, particularly the enzyme catalyzes the reaction between at least two molecules, wherein at least one of the molecules is derived from said sample. For example, a positive reaction may be a change in color of a solution or the precipitation of a molecule, hence the formation of a solid in a solution.

A method for the diagnosis of diseases or disorders or a certain medical condition in a subject to be tested may comprise the steps:
a) obtaining a sample from said subject;
b) obtaining a sample from a healthy subject as negative control reference;
c) obtaining a sample from a subject suffering from this disease as positive control reference;
d) determining the amounts of a particular molecule present in each of the samples obtained in steps a) to c);
e) comparing the amounts of said molecule present in each of the samples obtained in step d);

### Description of the figures

**Figure 1A****)** Schematic view of the process for the production of a protected enzyme on a solid carrier material;
a) the enzyme is bound on a solid carrier material;
b) a protecting layer grows around the immobilized catalyst;
c) over time the protecting layer can completely surround the enzyme.

**Figure 1B****)** Schematic representation of the enzyme protection strategy;
a: enzyme (circular shape) immobilization on the solid silica support (black);
b: self-assembly of the protection layer building blocks around the enzyme, c&d: protection layer growth (grey).

**Figure 2A****)** SEM micrograph of the silica nanoparticles (SNPs) used as carrier material.
**Figure 2B****)** SEM micrographs of the enzyme-immobilized SNPs

| | |
|---|---|
| after | 4 (left), |
| | 6 (middle) |
| and | 20 hours |

of protective layer growth
**Figure 3****:** Protective layer thickness

| | |
|---|---|
| measured after | 4, 6 and 20 hours of reaction |

**Figure 4****:** Relative activities of
free (black squares)
and
protected (white squares) enzymes heat-stressed at 42°C for increasing periods of time;
(activity values are normalized with the initial activity values measured without heat stress.)
**Figure 5****:** Relative activities of
free (black squares)
and
protected (white squares)
enzymes measured at increasing reaction temperatures.
**Figure 6****:** Relative activities of
free (white bars)
and
protected (black bars)
enzymes incubated at different pH values and measured at pH 6.5;
(activity values are normalized
with the initial activity values.)
**Figure 7****:** Relative activities of
free (empty squares)
and
protected (full squares)
enzymes measured at different pH values;
(activity values are normalized with the maximum activity observed at pH 6.5)
**Figure 8****:** Relative enzyme activity
during the silane layer growth;
(activity values are normalized with the activity of the immobilized catalyst in the absence of protective layer.)
**Figure 9****:** Relative enzyme activity of
the protected (black squares)
and
reference free enzyme (empty squares)
at increasing heat stress durations (65°C);
(activity values are normalized with the activity of the immobilized catalyst before temperature stress)
**Figure 10****:** Protective layer thickness measured at increasing reaction times for another example according to the invention
**Figure 11****:** Relative activities of
free lactase
and
lactase protected
with a layer made of APTES-TEOS mixture or
with a layer made of silane mixture
heat-stressed at 50°C for one hour.

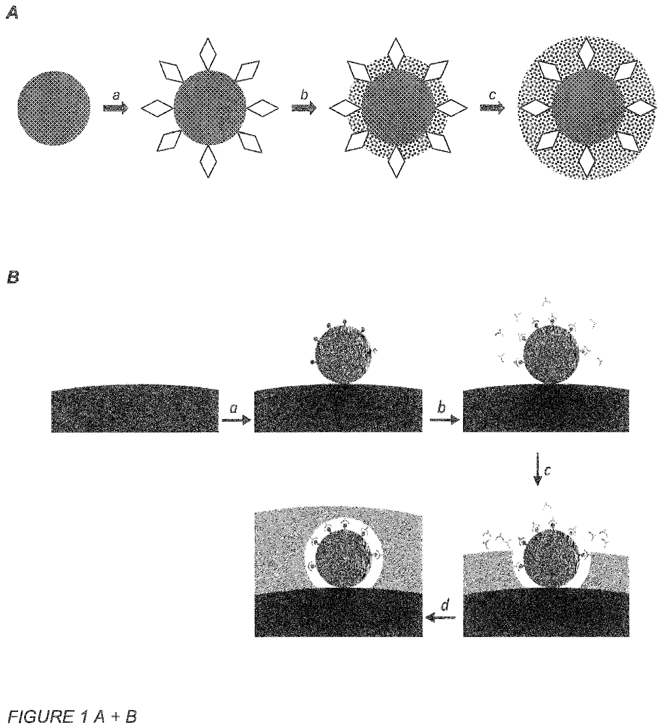

According to Figure 1 A) showing a schematic view of the process for the production of a protected enzyme on a solid carrier material, the enzyme is first bound on a solid carrier material (compare step a)). Then, a protecting layer grows around the immobilized catalyst (compare step b)). Thereafter, over-time the protecting layer can completely surround the enzyme (compare step c)).

Then, Figure 1 B) shows a schematic representation of the enzyme protection strategy. First, the enzyme (circular shape) immobilization on the solid silica support (black, compare step a)).
Then, self-assembly of the protection layer building blocks around the enzyme takes place.
Then, compare steps c&d, the protection layer grows (grey). The environment around the enzyme (i.e. interactions between the enzyme outer surface and the cavities formed in the organosilica layer) provide a great conformational stabilization effect. In this example, the carrier material is silica (nanoparticle), and the protecting layer is organosilica (polysilsesquioxane) produced by the polycondensation reaction of silica precursors (tetraorthosilicate & organosilanes).
It is noted that the increased stabilization of the tertiary protein structure against stress achieved is concluded from activity measurements as only a properly folded enzyme is likely to be active.

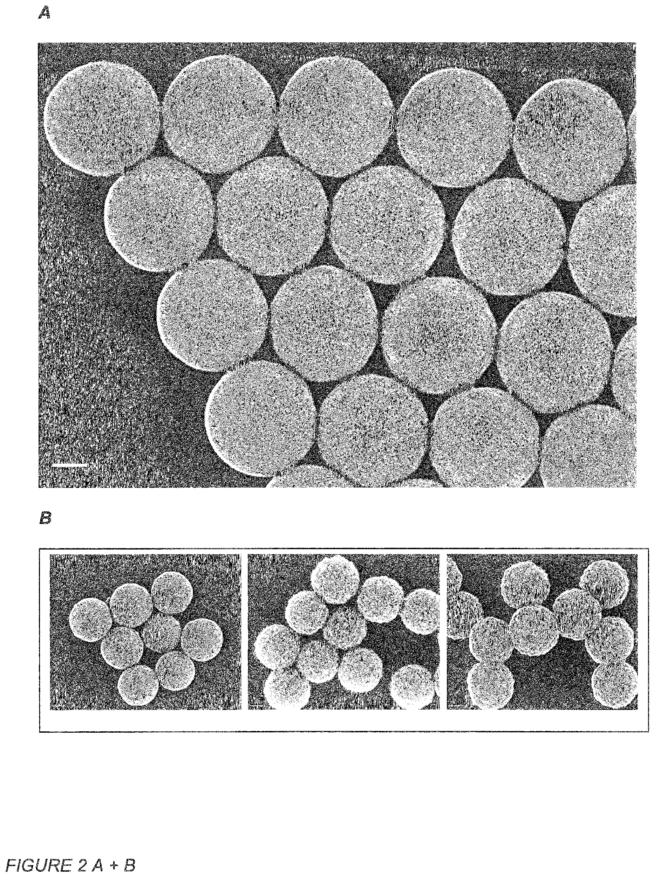

In Figure 2 A) SEM micrographs of the silica nanoparticles (SNPs) used as carrier material are shown, and SEM micrographs of the enzyme-immobilized SNPs after 4 (left), 6 (middle) and 20 hours of protective layer growth are shown in Fig. 2B.

### Examples

### Example 1: Lactase immobilization on a solid carrier material and protection by an organo-silica (i.e. silsesquioxane) layer

Lactase/β-galactosidase (EC 3.2.1.23) immobilization on a solid carrier material such as silica nanoparticles (SNPs) and protection involves four main steps that are:
i. Surface modification of the SNPs in order to introduce anchoring points (*i.e.* amine) for the further chemical coupling with the enzyme
ii. Chemical reaction of the introduced amine moieties with a bi-functional cross-linker (e.g. glutaraldehyde)
iii. Enzyme coupling at the surface of the SNPs through the free active functions of the bi-functional cross-linker
iv. Polycondensation of silane building-blocks around both immobilized enzymes and free surface of the SNPs to yield a protective layer.

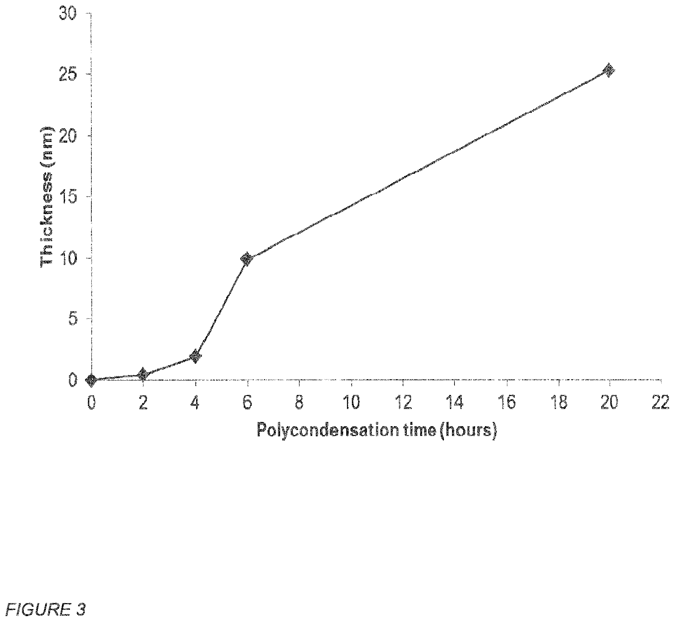

This synthetic procedure allows producing a protective layer at the surface of the SNPs surrounding and thus protecting the enzyme. The thickness of the produced protective layer can be adjusted by design, depending on the targeted application.
i) SNPs were produced using the conventional Stöber method adapted from the report of Imhof et al. (J. Phys. Chem. B 1999, 103, 1408), as follows. Ethanol (345.4 ml), ammonia 25% (39.3 ml) and TEOS (tetraethylorthosilicate, 15.3 ml) were mixed in a round bottom flask and this mixture was stirred at 600 rpm during 20 hours, at a constant temperature of 20°C. The resulting precipitate was consequently washed twice with ethanol and twice with water, and freeze-dried to yield bare SNPs that were characterized using scanning electron microscopy (Zeiss, SUPRA 40 VP). The acquired micrographs were used for particle size measurement using the analysis® (Olympus) software package (statistical analysis carried out on 100 measurements). In Fig. 2A is given a representative micrograph of the produced SNPs.
ii) In order to introduce, at the surface of the SNPs, amine functions that allowed the further anchoring of the to-be-protected enzyme, they were reacted with an amino-silane. It is important to note that this modification should be only partial so as to leave silanol groups for the further attachment of the protective layer.
   In more details, SNPs in suspension in water (18 mL; 3.2 mg/ml) were incubated with APTES (3-aminopropyltriethoxysilane, 11 mg) during 90 minutes at 20°C. After two washing steps in water, the resulting amino-modified SNPs were reacted during 30 minutes with a bi-functional cross-linker (to allow the further immobilization of the enzyme), glutaraldehyde, at a final concentration of 1 g/L.
iii) After two washing steps in water, the resulting SNPs were re-suspended in a MES (2-(N-morpholino) ethanesulfonic acid) buffer (pH 6.2, 1 mM, 5 mM MgCl₂) and incubated for 1 hour at 20°C with the enzyme, Lactase, (100 µg/ml) under magnetic stirring at 400 rpm.
iv) The protection of the enzyme immobilized on SNPs was carried out by incubating the produced enzyme-immobilized SNPs with a mixture of silane building blocks that self-assembled around the enzyme and underwent a polycondensation reaction that created a protecting layer around the enzyme. Adequate building blocks have to be selected in dependence from the protein or protein-type compound of interest and its amino acid residues on the surface. Examples for a preferred selection of self-assembling building blocks are given in TABLE 1, which provides a list of putative protective layer building blocks and the main forces, which interact between the protein surface's amino acid residues of the protected protein and the self-assembled building block. The polycondensation reaction also occurred at the bare surface of the SNPs allowing the attachment of this layer at the surface of the SNPs. To that end, enzyme-immobilized SNPs (18 mL; 3.2 mg/ml) were first reacted at 20°C under stirring at 400 rpm with 36 µl of TEOS. After 2 hours of reaction, 18 µl of APTES were added and the protective layer was allowed to grow over time at 4°C. Samples of SNPs were collected every 2 hours and the reaction was stopped after 20 hours by two washing steps in MES buffer. The protective silane layer thickness at different time points was measured as described above and is reported in Fig. 2 B and Fig. 3. From these results, it could be seen that the organosilane layer is 2, 10 and 25 nm thick after respectively 4, 6 and 20 hours of reaction. These results confirmed the possibility to control the organosilica layer growth at the surface of enzyme-immobilized SNPs.

The enzymatic activity of the so-produced particles was assayed using *ortho*-nitrophenyl-β-galactoside (ONPG) as artificial substrate and following spectrophotometrically the appearance of the product *ortho*-nitrophenol (ONP) at 420 nm revealed in alkaline conditions. In more details, SNPs were collected at increasing durations of silane polycondensation and washed twice in MES buffer. To measure the lactase activity, SNPs were incubated for 5 minutes at 40°C with ONPG (40 mM) at pH 6.5 and the reaction was stopped by the addition of an equal volume of an aqueous solution of Na₂CO₃ (1 M). The result showed that 45 % of the initial enzymatic activity was present on the particles possessing a protective layer of 25 nm confirming that even when the enzyme is buried into an organosilica protective layer, it maintains partially its activity.

With respect to Fig. 3, it is noted that the layer thickness obtained over time depends on the kinetics of the polycondensation reaction and correspondingly will depend on the (i) time, (ii) temperature and (iii) mixture of organosilanes used. For (i) it is clear that the longer the reaction is kept, the thicker the protective layer will be. For the temperature, it will be understood that the higher the temperature, the faster the kinetics and therefore the thicker the protective layer (and vice versa). With respect to the mixture of monomers, it is noted that the presence of organosilanes with basic character (APTES or UPTES) will boost the protective layer growth.

In Fig. 3, an initial delay can be observed which currently is attributed to an initial pre-hydrolysis and solubilization of the more hydrophobic monomers. Once this initial reactions are done, the thickness of the protective layer becomes measurable (e.g. by scanning electron micrographs and statistical analysis of the particle size with a software). If the protective layer has reached the thickness needed and the reaction and thus further thickening of the protective layer shall be stopped, this can be done in case of a protective layer building polycondensation reaction by washing the particles and removing the unreacted monomers.

**Table 1: List of protection layer building blocks and the main forces interacting between these building blocks and the amino acid residues on the surface of the protein or protein-type compound of interest, which is embedded in the protective layer. The selection of the adequate building blocks should be adapted to the present protein surface amino acid residues.**

| **Protection layer building block** | **Protein surface amino acids (3-letter-code)** | **Main interactions involved** |
|---|---|---|
| Benzyltriethoxysilane | Phe, Tyr, Trp | p-p (aromatic) interactions |
| Propyltrimethoxysilane | Gly, Ala, Leu, Ile, Val, Pro | van der Waals |
| Isobutyltriethoxysilane | Gly, Ala, Leu, Ile, Val, Pro | van der Waals |
| n-Octyltriethoxysilane | Gly, Ala, Leu, Ile, Val, Pro | van der Waals |
| Hydroxymethyltriethoxysilane | Ser, Thr, Asp, Glu, Asn, Gln, Tyr | H-bonding |
| Bis(2-hydroxyethyl)-3-aminopropyltriethoxysilane | Ser, Thr, Asp, Glu, Asn, Gln, Tyr | H-bonding |
| Aminopropyltriethoxysilane | Asp, Glu | Ionic |
| T etraorthosilicate | Lys, Arg, His | Ionic |
| Carboxyethylsilanetriol | Lys, Arg, His | Ionic |

With respect to Table 1, the following is noted: First, while Table 1 is given in connection with the embedding of a specific functional constituent, it will be understood that the infor-mation disclosed by Table 1 and in connection therewith will be relevant to other functional constituents as well. Then, it will be understood by the average skilled person that the list of Table 1 is not exhaustive and that there are other organosilane monomers that can be used for the method according to the invention.

In this context, it is further noted that the list is not exhaustive as it would be difficult to establish an exhaustive one e.g. as non-commercial silanes could also be produced and used. Furthermore, on certain occasions, it may be advantageous to use organosilanes carrying large and bulky groups, e.g. octadecyltrimethoxysilane and triphenyl-triethoxysilane, e.g. to obtain sufficiently large pores.

Additionally, it will be noticed that the silanes in the table as well as frequently throughout other parts of the disclosure are given as triethoxy derivatives; yet, referring to a single derivative rather than all possible derivatives such as e.g. tri-methoxy or tri-hydroxyethoxy derivatives has been done to simplify reading and to simultaneously direct the reader to organo silanes readily available, not in order to restrict the scope of the disclosure. The average skilled person will understand that reference could be had to "silanes" in general (e.g. aminopropyl silane instead of referring to the aminopropyltriethoxy silane in the table). Furthermore, the list is not even complete with respect to silanes particularly relevant for specific main interactions. As an example, Ureidopropyltriethoxysilane and (N-Acetylglycyl)-3-aminopropyltrimethoxysilane, could be included as further strong H-bonding donor acceptor monomers.

Then, it is noted that CYS and MET are not listed in Table 1. However, if these are present at the surface of the functional constituent, it is possible to select appropriate organosilanes that interact with these amino acids. For example, use can be made of the fact that these amino acids can form covalent disulfide bridges (-S-S-) to suitable organosilanes bearing an -SH group, such as (3- Mercaptopropyl)trimethoxysilane or, in a more generic way (3-Mercaptopropyl) silane. Hence, e.g. organo silanes bearing a functional -SH group could also be added to the list.
Finally, it will be obvious that Table 1 will not only be referred to with respect to encapsulation of Lactase immobilization but will be found instructive by the average skilled person intending to embed other functional constituents as well,

### Example 2: Protection against thermal stress

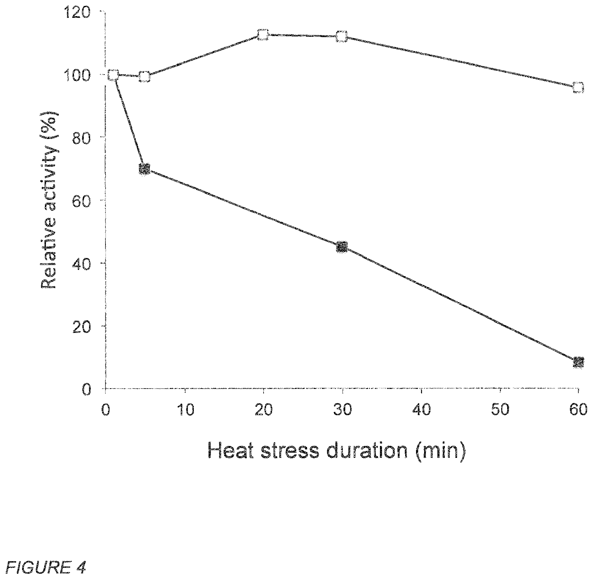

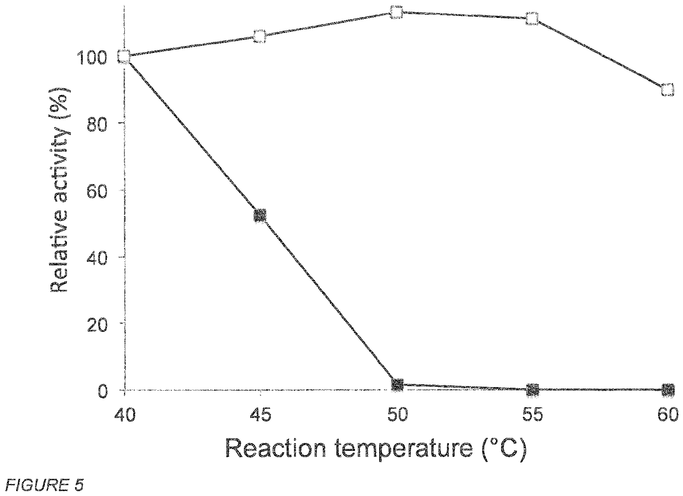

The thermal resistance of enzymes protected with the method described herein was tested using lactase-modified particles with a protective layer of 20 nm, produced as described in Example 1. The catalytic activities were measured using the ONPG colorimetric method also described in example 1.
First the protected and free enzymes were thermally stressed at 42°C for increasing periods of time, and the activity measured; cf. Fig. 4.
It could be seen that while the activity of the free enzymes dropped down to 70% after 5 min, 45% after 30 min and 8% after 60 minutes; the protected enzyme remained for all the tested conditions at activity values higher than 90%. Interestingly, the activity even increased to 112% for 20 min and 30 min of heat stress. This set of results clearly demonstrated the advantage of the enzyme protection strategy described herein.
In addition, the enzyme activity was measured at increasing temperature values; the results are reported in Fig. 5.
From the results reported in Figure 5, it could be seen than the activity of the free enzyme dropped down to a value of 52% at 50°C, and no activity could be measured at 50, 55 and 60°C. For the protected enzyme, interestingly, the activity increased to 106%, 113% and 111% for reaction temperatures of 45°C, 50°C and 55°C. A slight decrease of 11% is observed for the reaction measured at 60°C.

### Example 3: pH resistance and pH range broadening of protected Lactases

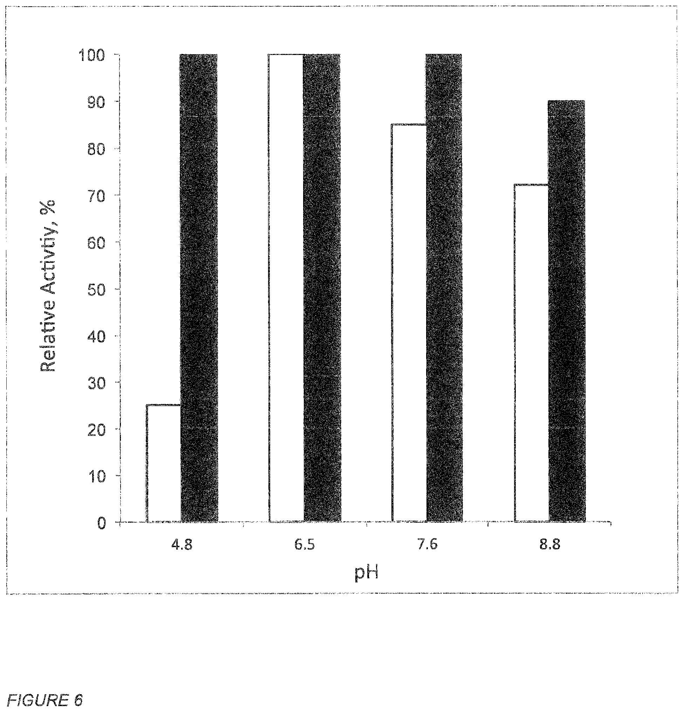

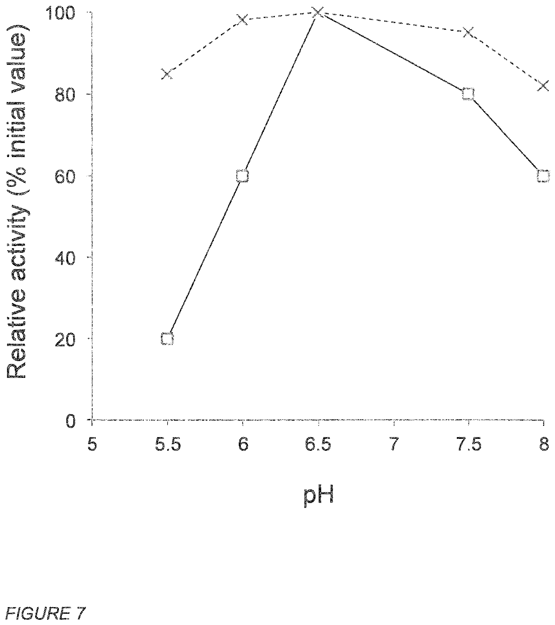

The resistance of enzymes protected with the method described herein and the broadening of their pH activity range was tested using lactase-modified particles with a protective layer of 20 nm; produced as described in example 1. The catalytic activities were measured using the ONPG colorimetric method also described in example 1
First, free and immobilized enzymes were incubated during 15 minutes at different pH values (4.8, 6.5, 7.6, 8.8); the pH value was then adjusted to the optimal catalytic pH (6.5) and the activity of the different systems measured; the results are reported in Fig. 6. It could be seen that while the treatment at pH 6.5 did affect neither the immobilized enzyme nor its free counterpart, a treatment at pH 4.8 caused a drastic decrease in activity of the free enzyme down to 25%, while the protected enzyme remained unaffected. At pH values of 7.6 and 8.8, the free enzyme lost 15% and 28% of activity respectively while the protected enzyme was unaffected at pH 7.6 and lost only 10% of activity at pH 8.8.
Additionally, catalytic activities of free and immobilized lactases were measured using the ONPG colorimetric method described in example 1 at different pH values (5.5, 6.0, 6.5, 7.5 and 8.0). The relative activity values measured are reported in figure 7.
Both enzymatic systems had an optimal pH value of 6.5. Increasing the pH to 7.5 and 8.0, the free enzyme showed a decay in activity of 20% and 40% respectively, while the protected enzyme lost only 5% and 18% in the same conditions. For acidic pH values, the free enzyme lost 40% and 80% of activity for pH value of 6.0 and 5.5, respectively; while the protected enzyme lost only 2% and 15%. Those results confirmed the protection of the enzyme resulting in the broadening of its activity range.

### Example 4: Protection against protease attack

The resistance to proteases of enzymes protected with the method described herein was tested using lactase-modified particles with a protective layer of 20 nm, produced as described in example 1. Free and protected enzymes were incubated with proteinase K and trypsin (1 mg/mL) for 60 min at 37 °C in 0.1 M Tris-HCl (pH 7.4). While the activity of the free enzyme dropped down to zero, the activity of the protected enzyme remained unchanged.

### Example 5: Acid phosphatase immobilization on SNPs and protection by an organo-silica (i.e. silsesquioxane) layer, and temperature stress test.

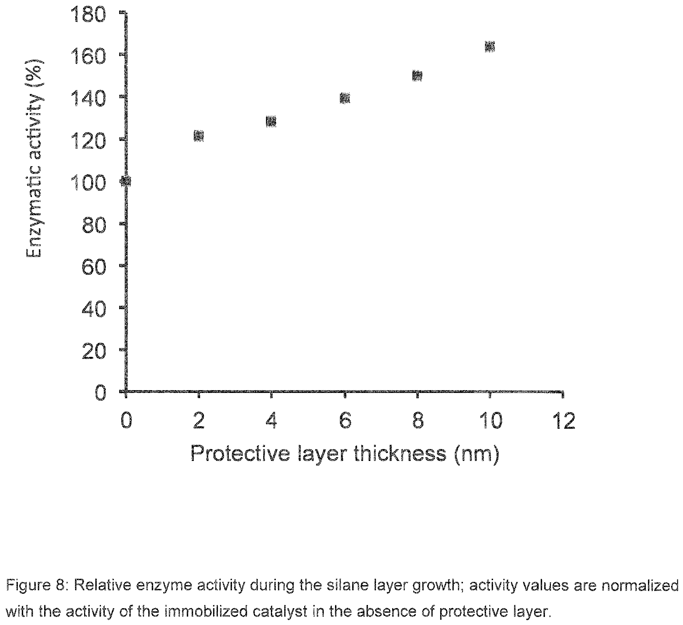

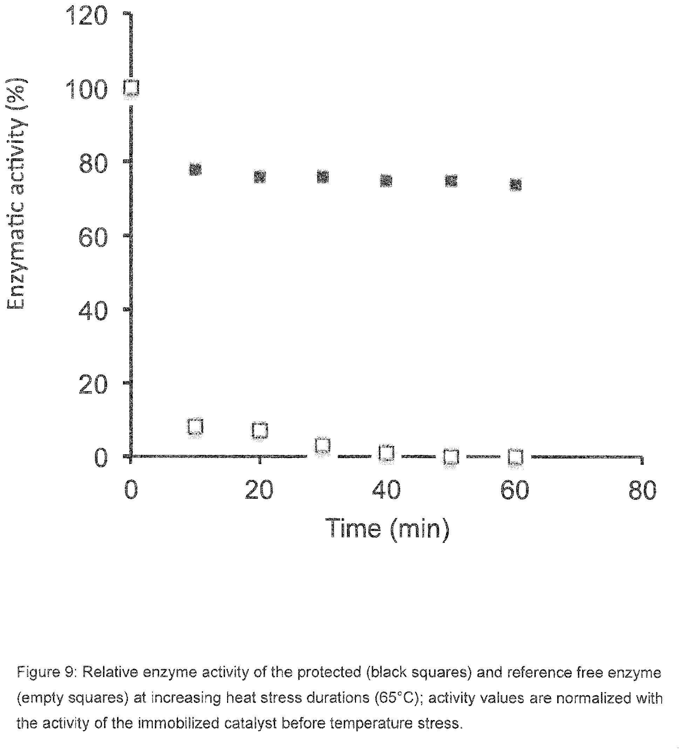

Acid phosphatase (EC 3.1.3.2) immobilization on SNPs and protection by growing a layer of organosilanes have been performed as described in example 1. Protected catalysts, with increasing protection layer thicknesses, were produced and assayed using *para-*nitrophenylphosphate (*p*NPP) as artificial substrate. The appearance of the product *p-*nitrophenol (*p*NP) at 405 nm was followed spectrophotometrically and revealed in alkaline conditions.
Briefly, to measure the acid phosphatase activity, the protected biocatalysts were incubated for 5 minutes at 37°C with *p*NPP (15 mM) at pH 4.8 and the reaction was stopped by the addition of an equal volume of an aqueous solution of NaOH (100 mM); the results are given in Figure 8. It is shown that the enzymatic activity does increase with the presence of the protective layer.
The resistance to temperature was assayed by incubating the produced particles (and soluble reference enzyme) at 65°C for increasing durations. The results of activity are reported in Figure 9. It is demonstrated that while the free reference enzyme lost more than 90% of activity after 10 minutes and more than 95% after 30 minutes, the protected enzyme maintains as much as 80% after 10 minutes of treatment and more than 75% after 60 minutes.

### Example 6: Lactase immobilization on SNPs and protection by a layer made of a silane mixture

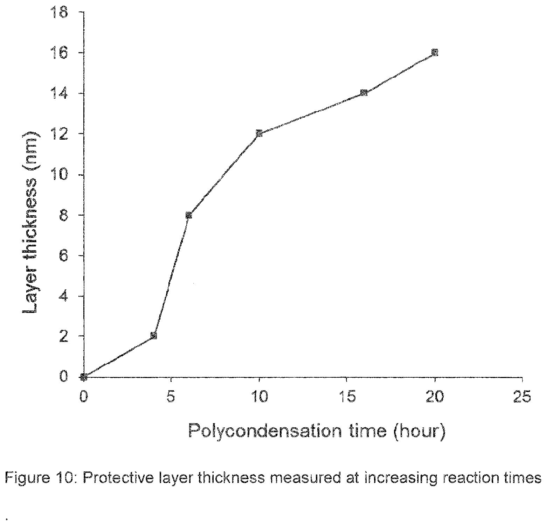

The Lactase/β-galactosidase (EC 3.2.1.23) immobilization on SNPs has been performed as described in example 1. The protection of the enzyme immobilized on SNPs was carried out by incubating the produced enzyme-immobilized SNPs with a mixture of silane building blocks that self-assembled around the enzyme and underwent a polycondensation reaction that created a protecting layer around the enzyme. The used silanes were: APTES, TEOS, benzyltriethoxysilane (BTES), Propyltrimethoxysilane (PTES), and Hydroxymethyltriethox-ysilane (HMTES). In more details, enzyme-immobilized SNPs (18 mL; 3.2 mg/ml) were first reacted at 20°C under stirring at 400 rpm with 36 µl of TEOS. After 1 hour of reaction, 18 µl of APTES, 18 µl BTES, 18 µl PTES and 36 µl HMTES were added and the protective layer was allowed to grow at 20°C. Samples of SNPs were collected at increasing reaction times and the reaction was stopped after 20 hours by two washing steps in MES buffer. The protective silane layer thickness, at different time points, was measured as previously described. As shown in Fig. 10, the organosilane layer was 2, 8, 12 and 16 nm thick after 4, 6, 10, 17 and 20 hours of reaction, respectively.

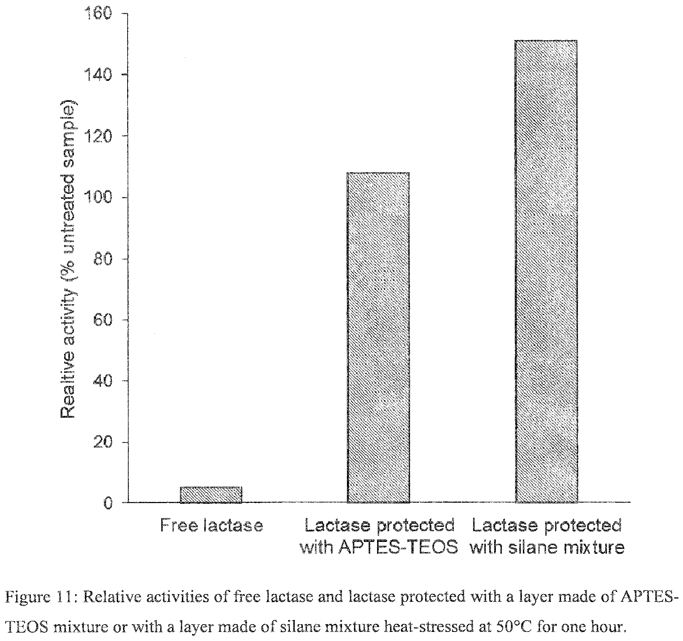

The thermal resistance of the so-produced protected lactase was tested by incubation at 50°C for 60 min and compared to catalyst protected using a mixture of APTES-TEOS as shown in figure 5. The result showed that while the activity of the free lactase was lower than 5 % after 1 hour treatment at 50°C, the activity of the lactase protected with a layer made of APTES-TEOS or made of silane mixture was higher than 110 % and 150 % respectively (Figure 11).

It is noted that the present invention claims priority of EP 13 17 850.4. The priority giving document is fully enclosed herein for purposes of disclosure.

Accordingly, what has been described above inter alia is a composition comprising at least one protein or protein-type compound and optionally further comprising at least one molecule selected from the groups of adaptor molecules, anchoring molecules, scaffold molecules and/or receptor molecules, immobilized at the surface of a solid carrier, wherein said protein or protein-type compound and the at least one optional molecule is fully or partially embedded in a protective material comprised of self-assembling building blocks, which building blocks comprise functional groups, which interact with the chemical groups of the protein or protein-type compound and the at least one optional molecule such that a porous nano-environment is established on the carrier surface and around the immobilized protein or protein-type compound and the at least one optional molecule which stabilizes the native conformation and preserves the function of the protein or protein-type compound and the at least one optional molecule.

Furthermore, it has been suggested that in such a composition the solid carrier is a nanoparticle, particularly a silica nanoparticle (SNP), particularly a gold nanoparticle, particularly a titanium nanoparticle.
Furthermore, it has been suggested that in such a composition the binding of the protein or protein-type compound to the surface of the solid carrier is covalent binding.
Furthermore, it has been suggested that in such a composition the size of the nanoparticle is in a range of between 20 and 1000 nm, particularly of between 200 and 500 nm., particularly between 300 and 400 nm.
Furthermore, it has been suggested that in such a composition the thickness of the protective material ranges from 1 to 100 nm, 1 nm to 50 nm, 1 nm to 30 nm, 1 nm to 25 nm, 1 nm to 20 nm, 1 nm to 15 nm, preferably 5 nm to 15 nm.
Furthermore, it has been suggested that in such a composition the self-assembled protective material has a pore size which allows the diffusion of substrates, particularly a pore size of between 1 nm and 10 nm, particularly between 2 nm and 9 nm, particularly between 3 nm and 8 nm, particularly between 4 nm and 7 nm, particularly between 4 nm and 6 nm, particularly between 4 nm and 5 nm.
Furthermore, it has been suggested that in such a composition the functional groups of the self-assembling protective material are groups interacting with the amino acid side chains of the protein or protein-type compound, particularly based on weak force interactions. Furthermore, it has been suggested that in such composition **said protective material is or-ganosilica.**
Furthermore, it has been suggested that said protein or protein-type compound is an enzyme or enzyme-type compound, particularly an enzyme or enzyme-type compound, which is selected from the group consisting of oxidoreductases, transferases, hydrolases, lyases, isomer-ises and/or ligases.
Furthermore, it has been suggested that in such a composition said protein or protein-type compound and/or at least one of the optional molecules is bound to the surface of the solid carrier by a bi-functional cross-linker, particularly a bi-functional cross-linker selected from the group of glutaraldehyde, disuccinimidyl tartrate, bis[sulfosuccinimidyl] suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, activated sulfhydryls (e.g. sulfhydryl-reactive 2-pyridyldithio).
Furthermore, it has been suggested that in such a composition the protective material provides protection to:
a) a pH, different from the optimal pH of the at least one protein or protein-type compound, wherein the pH value differs from the optimal pH by a value of +/- 5, +/- 4; +/- 3, +/- 2, +/- 1, +/- 0.5 pH units; and/or
b) chemical stresses; and/or
c) biological stresses; and/or
d) solvents; and/or
e) physical stress; and/or
f) elevated temperatures, which exceed the optimal temperature for the unprotected protein or protein-type compound by 60°C, particularly by 50°C, particularly by 40°C, particularly by 30°C, particularly by 20°C, particularly by 10°C, particularly by 5°C; and/or
g) reduced temperatures, which deviate from the optimal temperature for the unprotected protein or protein-type compound by 60°C, particularly by 50°C, particularly by 40°C, particularly by 30°C, particularly by 20°C, particularly by 10°C, particularly by 5°C.
Furthermore, it has been suggested that in such a composition said immobilized and protected enzyme has:
a) an increased activity under stress conditions when compared to the free-unprotected enzyme; and/or
b) an increased recoverability for use in continuous operation compared to the free-unprotected enzyme.
Then, a method for producing such a composition according is suggested comprising the steps of:
a) obtaining a solid carrier; and
b) immobilizing at least one protein or protein-type compound of interest, particularly at least one enzyme or enzyme-type compound, and, optionally, at least one optional molecule at the surface of the carrier; and
c) incubating the at least one protein or protein-type compound and, the optional molecule bound at the surface of the solid carrier with self-assembling building-blocks to yield a porous nano-environment around the free surface of the solid carrier and the at least one protein and/or protein-type compound and optional molecule bound at the surface of the solid carrier, and
d) stopping the self-assembly reaction of the protective material at a specific time point to obtain a preferred protective layer with a desired thickness.
Furthermore, it has been suggested that such a composition be used in a catalytic process. Furthermore, it has been suggested that such a composition be used in therapy, for example, of sphingomyelinase deficiency (ASMD) syndrome, Niemann-Pick Disease (NPD), lysosomal storage diseases, Gaucher disease, Fabry disease, MPS I, MPS II, MPS VI and Glycogen storage disease type II, cancer, allergic diseases, metabolic diseases, cardiovascular diseases, autoimmune diseases, nervous system disease, lymphatic disease and viral disease.

### References

1. C. Mateo et al., Enzyme Microb. Technol, 2007, 40, 1451
2. D. Brady and J. Jordaan, Biotechnol Lett, 2009, 31, 1639
3. R.C. Rodrigues et al., Chem Soc Rev, 2013 [Epub ahead of print]
4. H. R. Luckarift et al., Nat Biotechnol, 2004, 22, 211
5. U. Hanefeld et al., Chem Soc Rev, 2009, 38, 453
6. M. Hartmann and D. Jung, J Mater Chem, 2010, 20, 844
7. L. Hilterhaus et al., Bioproc Biosyst Eng, 2008, 31, 163
8. R. R. Naik et al., US2005095690; 2005
9. S.A. Werchant, WO2010039384; 2010
10. W. R. K. Schoevaart, US 20120149082A1; 2012
11. Ostaszewski et al., US 2010/0240116 A1; 2010
12. R. Bond et al., US 7642077B2, 2010
13. R. Bond et al., WO2005056808, 2006
14. S. J. Chung et al., US 2010/0196937A1, 2010
15. J.H. Chang et al., US 2012/0264188A1, 2012
   A. Auger et al., WO2011/058046A1, 2011
   A. Auger et al., US 2012/0283379A1, 2012
16. A Leech et al., WO 2011/060129 A1, 2011
17. N. J. Lant et al., WO 2013/003025, 2013
18. L.S. Wong et al., Chem Rev, 2009, 109, 4025
19. S. C. Esener et al., WO2012142625 A2, 2012
20. Q. Husain, Crit Rev Biotechnol, 2010, 30, 41
21. H. Hirohara et al., EP0026672A2, 1981
   B. Giacomini et al., J Mol Catal B-Enzym, 1998, 4, 313
   A. Tanriseven and S. Dogan, Process Biochem, 2002, 38, 27
22. E.J. Mammarella and A.C. Rubiolo, J Mol Catal B Enzym. 2005, 34, 7
23. S. Rejikumar and S. Devi, Int J Food Sci Technol, 2001, 36, 91
   I. Roy and M. N. Gupta, Process Biochem, 2003, 39, 325
24. N. Albayrak and S.T. Yang, Enz. Microb Technol, 2002, 31, 371
25. N. Albayrak and S. T. Yang, Biotechnol Prog, 2002, 18, 240
   C. Mateo et al., Biotechnol Prog, 2004, 20, 1259
26. M. Ladero et al., Enzyme Microb Tech, 2000, 27, 583
27. L. Betancor et al., Biotechnol Bioeng, 2008, 99, 261
28. Y. Kuwahara et al., Chem Commun, 2012, 48, 2882
29. Z. Grosova et al, Biotechnol Lett. 2008, 30, 763
30. D.F.M. Neri et al., Catal Commun, 2008, 9, 2334
**31**. J. Hotchkiss and J. N. Talbert, US 2010196985A1, 2010
32. WO 93/07263 A2 (GENENCOR INT [US]) 15 April 1993 (1993-04-15)
33. US 6 268 329 B1 (MARKUSSEN ERIK KJ AELIG R [DK]) 31 July 2001 (2001 - 07-31)
34. YONG-GINO XIA ET AL: "Protein recognition onto silica particles using chitosan as intermedium substrate", JOURNAL OF BIOMEDICAL MATERIALSRESEARCH PART A, vol: 90A, no. 2, 1 August 2009 (2009-08-01), pages 326-332, XP055099686, ISSN: 1549-3296, DOI: 10.1002/jbm.a.32084
35. MATEO ET AL: "Improvement of enzyme activity, stability and selectivity via immobilization techniques" ENZYME AND MICROBIAL TECHNOLOGY, STONEHAM, MA, US, vol. 40, no. 6, 29 March 2007. (2007-03-29), pages 1451-1463, XP022003968, ISSN: 0141 -0229, DOI: 10.1oie/J.ENZMICTEC.2007.01.018
36. ULF HANEFELD ET AL: "Understanding enzyme immobilization", CHEMICAL SOCIETY REVIEWS, vol. 38, no. 2, 1 January 2009 (2009-01-01), page 453, XP055099160, ISSN: 0306-0012, DOI: 10.1039/b711564b
37. DEAN BRADY ET AL: "Advances in enzyme immobilisation", BIOTECHNOLOGY LETTERS, SPRINGER NETHERLANDS, DORDRECHT, vol. 31, no. 11, 10 July 2009 (2009-07-10), pages 1639-1650, XPO19746235, ISSN: 1573-6776, DOI: 10.1007/S10529-009-0076-4
38. GALLIKER P ET AL: "Laccase-modified silica nanoparticles efficiently catalyze the transformation of phenolic Compounds", JOURNAL OF COLLOID AND INTERFACE SCIENCE, ACADEMIC PRESS, NEW YORK, NY, US, vol. 349, no. 1, 1 September 2010 (2010-09-01), pages 98-105, XP027113832, ISSN: 0021-9797 [retrieved on 2010-05-16]
39. CHIORCEA-PAQUIM A M ET AL: "AFM nanometer surface morphological study of in situ electropolymerized neutral red redox mediator oxysilane sol-gel encapsulated glucose oxidase electrochemical biosensors", BIOSENSORS AND BIOELEC-TRONICS, ELSEVIER BV, NL, vol. 24, no. 2, 15 October 2008 (2008-10-15), pages 297-305, XP023439562, ISSN: 0956-5663, DOI: 10.1016/J.BIOS.2008.04.001 [retrieved on 2008-04-1 1]
40. WO 2005/056808 A2 (GENENCOR INT [US]; DOW CORNINGI [US]; BOND RISHA [US]; JEWETT MICHAEL C) 23 June 2005 (2005-06-23)
41. SANJIB BHATTACHARYYA ET AL: "Polymer-coated mesoporous silica nanoparticles for the controlled release of macromolecules", ACTA BIOMATERIALIA, vol. 8, no. 9, 1 September 2012 (2012-09-01), pages 3429-3435, XP055099688, ISSN: 1742-7061, DOI: 10.1016/j.actbio. 2012.06.003

## Claims

1. A method of producing a composition, the composition comprising at least
a solid carrier,
a functional constituent, selected from a protein and a protein-type compound, wherein the protein and the protein-type compound is an enzyme,
a protective layer for protecting the enzyme, by embedding the enzyme at least partially, and
at least one bi-functional cross-linker to bind the enzyme to the surface of the solid carrier,
wherein the method comprises the steps that,
first the at least one enzyme is immobilized on the surface of the solid carrier,
and then the protective layer for protecting the enzyme by at least partially embedding the enzyme, is built with building blocks at least part of which are monomers capable of interacting with each other and the immobilized enzyme, wherein the building blocks build the protective layer by a poly-condensation of silica precursors under aqueous conditions,
wherein the silica precursors are tri-alkoxy-silane and tetra-alkoxy-silane, and,
wherein the method comprises the further step of after a specific reaction time interval stopping a self-assembly reaction of the protective material for building the protective layer with the building blocks to so obtain a preferred protective layer with a desired thickness,
wherein the thickness of the protective layer ranges from 1 to 25 nm and is between 50% and 150% of the length of the longer axis of the at least one enzyme.

2. A method according to claim 1, wherein the immobilized and protected enzyme has a) an increased activity under stress conditions when compared to the free-unprotected enzyme; and/or b) an increased recoverability for use in continuous operation when compared to the free-unprotected enzyme.

3. A composition comprising
a solid carrier,
at least one functional constituent, selected from a protein and a protein-type compound, wherein the protein and the protein-type compound is an enzyme,
at least one bi-functional cross-linker to bind the enzyme to the surface of the solid carrier and wherein the enzyme is immobilized on the surface of the solid carrier,
and a protective layer for protecting the enzyme by at least partially embedding the enzyme,
wherein the protective layer for protecting the enzyme is a layer built with building blocks, monomers of which are capable of interacting with each other and the immobilized enzyme,
wherein the building blocks build the protective layer by a poly-condensation of silica precursors under aqueous conditions, wherein the silica precursors are tri-alkoxy-silane and tetra-alkoxy-silane, and wherein the thickness of the protective layer ranges from 1 to 25 nm and is between 50% and 150% of the length of the longer axis of the at least one enzyme.

4. The composition of claim 3, wherein the immobilized and protected enzyme has a) an increased activity under stress conditions when compared to the free-unprotected enzyme; and/or b) an increased recoverability for use in continuous operation when compared to the free-unprotected enzyme.

5. The composition of claim 3, wherein the solid carrier is a nanoparticle, particularly a nanoparticle selected from the group of organic nanoparticle inorganic nanoparticle organic - inorganic composite nanoparticle, self-assembling organic nanoparticle, mesoporous silica nanoparticle (SNP), gold nanoparticle, titanium nanoparticle.

6. The composition of claim 3 or 5, wherein the carrier is a particulate carrier, in particular with a particle size up to 100µm, preferably in a range of between 20 and 1000 nm, particularly of between 200 and 500 nm, particularly between 300 and 400 nm.

7. The composition according to one of claims 3-6, wherein the thickness of the protective layer ranges from 1 nm to 20 nm, 1 nm to 15 nm, preferably 5 nm to 15 nm.

8. The composition according to one of claims 3-7, wherein said enzyme, is selected from the group consisting of oxidoreductases, transferases, hydrolases, lyases, isomerases and/or ligases.

9. The use of the composition of any one of the preceding claims in a catalytic process.

10. The use of the composition in a catalytic process of claim 9, wherein during the process the composition is subject to at least one of a pH different from the optimal pH of the functional constituent in particular such that the pH value differs at least by +/- 0.5 pH units and/or up to +/- 5 pH units from the pH optimal for the functional constituent and/or to chemical stresses; and/or to biological stresses; and/or to solvents; and/or to physical stress; and/or to elevated temperatures, which exceed the optimal temperature for the functional constituent by at least 5°C; and /or up to 60°C, particularly by 50°C, particularly by 40°C, particularly by 30°C, particularly by 20°C, particularly by 10°C, particularly by 5°C and/ or to reduced temperatures, which deviate from the optimal temperature for the functional constituent by at least 5°C; and /or up to 60°C.

11. The composition of any one of the preceding claims for use in therapy, in particular therapy of one of sphingomyelinase deficiency (ASMD) syndrome, Niemann-Pick Disease (NPD), lysosomal storage diseases, Gaucher disease, Fabry disease, MPS I, MPS II, MPS VI Glycogen storage disease type II, cancer, allergic diseases, metabolic diseases, cardiovascular diseases, autoimmune diseases, nervous system disease, lymphatic disease and viral disease.

## Patentansprüche

1. Verfahren zur Herstellung einer Zusammensetzung, wobei die Zusammensetzung mindestens
einen festen Träger,
einen funktionellen Bestandteil, ausgewählt aus einem Protein und einer proteinartigen Verbindung,
wobei es sich bei dem Protein und der proteinartigen Verbindung um ein Enzym handelt, eine Schutzschicht zum Schutz des Enzyms durch zumindest teilweise erfolgendes Einbetten des Enzyms, und
mindestens ein bifunktionelles Vernetzungsmittel, das das Enzym an die Oberfläche des festen Trägers bindet,
wobei das Verfahren die Schritte umfasst, dass zuerst das mindestens eine Enzym an die Oberfläche des festen Trägers immobilisiert wird,
und dann die Schutzschicht zum Schutz des Enzyms durch mindestens teilweise erfolgendes Einbetten des Enzyms mit Bausteinen aufgebaut wird, von denen mindestens ein Teil Monomere sind, die miteinander und mit dem immobilisierten Enzym in Wechselwirkung treten können, wobei die Bausteine die Schutzschicht durch eine Polykondensation von Silica-Vorläufern unter wässrigen Bedingungen aufbauen, wobei die Silica-Vorläufer Trialkoxysilan und Tetraalkoxysilan sind und
wobei das Verfahren den weiteren Schritt umfasst, nach einem spezifischen Reaktionszeitintervall eine Selbstassemblierungsreaktion des Schutzmaterials zum Aufbau der Schutzschicht mit den Bausteinen anzuhalten, so dass auf diese Weise eine bevorzugte Schutzschicht mit einer gewünschten Dicke erhalten wird, wobei die Dicke der Schutzschicht von 1 bis 25 nm reicht und zwischen 50% und 150% der Länge der längeren Achse des mindestens einen Enzyms liegt.

2. Verfahren nach Anspruch 1, wobei das immobilisierte und geschützte Enzym a) eine erhöhte Aktivität unter Belastungsbedingungen im Vergleich zu dem freien ungeschützten Enzym aufweist; und/oder b) eine erhöhte Rückgewinnbarkeit zur Verwendung bei kontinuierlichem Betrieb im Vergleich zu dem freien ungeschützten Enzym aufweist.

3. Zusammensetzung, umfassend
einen festen Träger,
mindestens einen funktionellen Bestandteil, ausgewählt aus einem Protein und einer proteinartigen Verbindung, wobei es sich bei dem Protein und der proteinartigen Verbindung um ein Enzym handelt,
mindestens ein bifunktionelles Vernetzungsmittel, das das Enzym an die Oberfläche des festen Trägers bindet, und wobei das Enzym an die Oberfläche des festen Trägers immobilisiert ist,
und eine Schutzschicht zum Schutz des Enzyms durch zumindest teilweise erfolgendes Einbetten des Enzyms,
wobei die Schutzschicht zum Schutz des Enzyms eine Schicht ist, die mit Bausteinen aufgebaut ist, deren Monomere miteinander und mit dem immobilisierten Enzym in Wechselwirkung treten können, wobei die Bausteine die Schutzschicht durch eine Polykondensation von Silica-Vorläufern unter wässrigen Bedingungen aufbauen, wobei die Silica-Vorläufer Trialkoxysilan und Tetraalkoxysilan sind und wobei die Dicke der Schutzschicht von 1 bis 25 nm reicht und zwischen 50% und 150% der Länge der längeren Achse des mindestens einen Enzyms liegt.

4. Zusammensetzung nach Anspruch 3, wobei das immobilisierte und geschützte Enzym a) eine erhöhte Aktivität unter Belastungsbedingungen im Vergleich zu dem freien ungeschützten Enzym aufweist; und/oder b) eine erhöhte Rückgewinnbarkeit zur Verwendung bei kontinuierlichem Betrieb im Vergleich zu dem freien ungeschützten Enzym aufweist.

5. Zusammensetzung nach Anspruch 3, wobei der feste Träger ein Nanopartikel ist, insbesondere ein Nanopartikel, ausgewählt aus der Gruppe der organischen Nanopartikel, anorganischen Nanopartikel, organischanorganischen Verbundnanopartikeln, selbstassemblierenden organischen Nanopartikeln, mesoporösen Silicananopartikeln (SNP), Goldnanopartikeln, Titannanopartikeln.

6. Zusammensetzung nach Anspruch 3 oder 5, wobei der Träger ein teilchenförmiger Träger ist, insbesondere mit einer Teilchengröße bis zu 100 µm, vorzugsweise in einem Bereich zwischen 20 und 1000 nm, insbesondere zwischen 200 und 500 nm, insbesondere zwischen 300 und 400 nm.

7. Zusammensetzung nach einem der Ansprüche 3 bis 6, wobei die Dicke der Schutzschicht von 1 nm bis 20 nm, 1 nm bis 15 nm, vorzugsweise 5 nm bis 15 nm, reicht.

8. Zusammensetzung nach einem der Ansprüche 3 bis 7, wobei das Enzym ausgewählt ist aus der Gruppe bestehend aus Oxidoreduktasen, Transferasen, Hydrolasen, Lyasen, Isomerasen und/oder Ligasen.

9. Verwendung der Zusammensetzung nach einem der vorhergehenden Ansprüche in einem katalytischen Verfahren.

10. Verwendung der Zusammensetzung in einem katalytischen Verfahren nach Anspruch 9, wobei die Zusammensetzung während des Verfahrens mindestens einer Bedingung unterworfen wird, eines pH-Werts, der vom optimalen pH-Wert des funktionellen Bestandteils abweicht, so dass sich insbesondere der pH-Wert mindestens um +/- 0,5 pH-Einheiten und/oder bis zu +/-5 pH-Einheiten von dem für den funktionellen Bestandteil optimalen pH-Wert unterscheidet; und/oder chemischen Belastungen; und/oder biologischen Belastungen; und/oder Lösungsmitteln; und/oder physikalischer Belastung; und/oder erhöhten Temperaturen, die die optimale Temperatur für den funktionellen Bestandteil um mindestens 5°C übersteigen; und/oder bis zu 60°C, insbesondere um 50°C, insbesondere um 40°C, insbesondere um 30°C, insbesondere um 20°C, insbesondere um 10°C, insbesondere um 5°C und/oder reduzierten Temperaturen, welche von der optimalen Temperatur für den funktionellen Bestandteil um mindestens 5°C abweichen; und/oder bis zu 60°C.

11. Zusammensetzung nach einem der vorhergehenden Ansprüche zur Verwendung bei der Therapie, insbesondere der Therapie einer Erkrankung von Sphingomyelinase-Mangel-(ASMD)-Syndrom, Niemann-Pick-Krankheit (NPD), lysosomalen Speicherkrankheiten, Morbus Gaucher, Morbus Fabry, MPS I, MPS II, MPS VI, Glykogen-Speicherkrankheit Typ II, Krebs, allergischen Erkrankungen, Stoffwechselerkrankungen, Herz-Kreislauf-Erkrankungen, Autoimmunerkrankungen, Erkrankungen des Nervensystems, lymphatischen Erkrankungen und Viruserkrankungen.

## Revendications

1. Méthode de production d'une composition, la composition comprenant au moins
un véhicule solide,
un constituant fonctionnel, choisi parmi une protéine et un composé de type protéine, où la protéine et le composé de type protéine est une enzyme,
une couche protectrice destinée à la protection de l'enzyme, par l'inclusion de l'enzyme au moins partiellement, et
au moins un agent de réticulation bifonctionnel destiné à fixer l'enzyme à la surface du véhicule solide,
où la méthode comprend les étapes dans lesquelles tout d'abord, la au moins une enzyme est immobilisée à la surface du véhicule solide,
puis la couche protectrice destinée à la protection de l'enzyme par l'inclusion de l'enzyme au moins partiellement, est construite à l'aide de synthons dont au moins une partie sont des monomères capables d'interagir les uns avec les autres et avec l'enzyme immobilisée, où les synthons construisent la couche protectrice par une polycondensation de précurseurs de silice dans des conditions aqueuses, où les précurseurs de silice sont le trialcoxysilane et le tétraalcoxysilane, et
où la méthode comprend l'étape supplémentaire consistant à, après un intervalle de temps de réaction spécifique, stopper une réaction d'auto-assemblage du matériau protecteur pour construire la couche protectrice avec les synthons de façon à obtenir une couche protectrice préférée ayant une épaisseur désirée, où l'épaisseur de la couche protectrice va de 1 à 25 nm et constitue entre 50% et 150% de la longueur de l'axe le plus long de la au moins une enzyme.

2. Méthode selon la revendication 1, dans laquelle l'enzyme immobilisée et protégée possède a) une activité accrue dans des conditions de stress par rapport à l'enzyme libre non protégée ; et/ou b) une capacité de récupération accrue pour une utilisation en fonctionnement continu par rapport à l'enzyme libre non protégée.

3. Composition comprenant
un véhicule solide,
au moins un constituant fonctionnel, choisi parmi une protéine et un composé de type protéine, où la protéine et le composé de type protéine est une enzyme,
au moins un agent de réticulation bifonctionnel destiné à fixer l'enzyme à la surface du véhicule solide, et où l'enzyme est immobilisée à la surface du véhicule solide,
et une couche protectrice destinée à la protection de l'enzyme, par l'inclusion de l'enzyme au moins partiellement,
où la couche protectrice destinée à la protection de l'enzyme est une couche construite à l'aide de synthons dont des monomères sont capables d'interagir les uns avec les autres et avec l'enzyme immobilisée, où les synthons construisent la couche protectrice par une polycondensation de précurseurs de silice dans des conditions aqueuses, où les précurseurs de silice sont le trialcoxysilane et le tétraalcoxysilane, et où l'épaisseur de la couche protectrice va de 1 à 25 nm et constitue entre 50% et 150% de la longueur de l'axe le plus long de la au moins une enzyme.

4. Composition selon la revendication 3, dans laquelle l'enzyme immobilisée et protégée possède a) une activité accrue dans des conditions de stress par rapport à l'enzyme libre non protégée ; et/ou b) une capacité de récupération accrue pour une utilisation en fonctionnement continu par rapport à l'enzyme libre non protégée.

5. Composition selon la revendication 3, dans laquelle le véhicule solide est une nanoparticule, en particulier une nanoparticule choisie dans le groupe constitué par les nanoparticules organiques, les nanoparticules inorganiques, les nanoparticules composites organiques-inorganiques, les nanoparticules organiques à auto-assemblage, les nanoparticules de silice mésoporeuses (SNP), les nanoparticules d'or, les nanoparticules de titane.

6. Composition selon la revendication 3 ou 5, dans laquelle le véhicule est un véhicule particulaire, en particulier doté d'une taille de particules allant jusqu'à 100 µm, préférablement dans la plage comprise entre 20 et 1000 nm, en particulier entre 200 et 500 nm, en particulier entre 300 et 400 nm.

7. Composition selon l'une des revendications 3-6, dans laquelle l'épaisseur de la couche protectrice va de 1 nm à 20 nm, de 1 nm à 15 nm, préférablement de 5 nm à 15 nm.

8. Composition selon l'une des revendications 3-7, dans laquelle ladite enzyme est choisie dans le groupe constitué par les oxydoréductases, les transférases, les hydrolases, les lyases, les isomérases et/ou les ligases.

9. Utilisation de la composition selon l'une quelconque des revendications précédentes, dans un processus catalytique.

10. Utilisation de la composition dans un processus catalytique selon la revendication 9, dans laquelle, lors du processus, la composition est soumise à au moins l'un parmi un pH différent du pH optimal pour le constituant fonctionnel, en particulier tel que la valeur de pH diffère d'au moins ± 0,5 unité de pH et/ou jusqu'à ± 5 unités de pH par rapport au pH optimal pour le constituant fonctionnel et/ou à des stress chimiques ; et/ou à des stress biologiques ; et/ou à des solvants ; et/ou à des stress physiques ; et/ou à des températures élevées, qui excèdent la température optimale pour le constituant fonctionnel d'au moins 5°C ; et/ou jusqu'à 60°C ; en particulier de 50°C, en particulier de 40°C, en particulier de 30°C, en particulier de 20°C, en particulier de 10°C, en particulier de 5°C, et/ou à des températures réduites, qui dévient de la température optimale pour le constituant fonctionnel d'au moins 5°C ; et/ou jusqu'à 60°C.

11. Composition selon l'une quelconque des revendications précédentes, destinée à une utilisation en thérapie, en particulier la thérapie de l'un parmi le syndrome de déficience en sphingomyélinase (ASMD), la maladie de Niemann-Pick (NPD), les maladies de stockage lysosomal, la maladie de Gaucher, la maladie de Fabry, la MPS I, la MPS II, la MPS VI, la maladie de stockage du glycogène de type II, le cancer, les maladies allergiques, les maladies métaboliques, les maladies cardiovasculaires, les maladies auto-immunes, les maladies du système nerveux, les maladies lymphatiques et les maladies virales.
